(19) Europäisches Patentamt
European Patent Office
Office européen des brevets

(11) **EP 2 956 493 B1**

(12) **EUROPEAN PATENT SPECIFICATION**

(45) Date of publication and mention
of the grant of the patent:
**04.01.2023 Bulletin 2023/01**

(21) Application number: **14705890.3**

(22) Date of filing: **12.02.2014**

(51) International Patent Classification (IPC):
**C08G 63/89** (2006.01)　　**C12P 7/62** (2022.01)

(52) Cooperative Patent Classification (CPC):
**B01D 11/02; C08G 63/89; C08K 3/014;
C08K 5/0016; C08K 5/005;** C12P 7/625

(86) International application number:
**PCT/IB2014/058943**

(87) International publication number:
**WO 2014/125422 (21.08.2014 Gazette 2014/34)**

(54) **PROCESS FOR THE EXTRACTION OF POLYHYDROXYALKANOATES FROM BIOMASS**

VERFAHREN ZUR EXTRAKTION VON POLYHYDROXYALKANOATEN AUS BIOMASSE

PROCÉDÉ D'EXTRACTION DE POLYHYDROXYALCANOATES (PHA) À PARTIR D'UNE BIOMASSE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **14.02.2013 US 201361764589 P**

(43) Date of publication of application:
**23.12.2015 Bulletin 2015/52**

(73) Proprietor: **Paques Biomaterials Holding B.V.
8561 EL Balk (NL)**

(72) Inventors:
• **WERKER, Alan Gideon
23442 Lomma (SE)**
• **JOHANSSON, Peter Stig Tomas
224 73 Lund (SE)**
• **MAGNUSSON, Per Olof Gösta
224 79 Lund (SE)**

(74) Representative: **Arnold & Siedsma
Bezuidenhoutseweg 57
2594 AC The Hague (NL)**

(56) References cited:
**EP-A1- 0 058 480　　EP-A2- 0 124 309
WO-A1-96/06179　　WO-A1-98/46782**

**Description**

TECHNICAL FIELD

This disclosure relates generally to the extraction and recovery of polyhydroxyalkanoates (PHAs) from biomass.

BACKGROUND

Today plastics are predominantly produced from petrochemical sources. Nevertheless, growing concern about natural resource availability and accumulation of plastic waste in nature motivates an ever-growing body of research and development focused on replacing petrochemical-based plastics with "bioplastics." Plastic material may be defined as a biaplastic if it is either biobased, biodegradable, or features both of these properties.

Polyhydroxyalkanoates (PHAs) represent a class of biobased polyesters that accumulate as intracellular granules in a wide variety of naturally occurring microorganisms. Methods of producing PHAs can include propagating pure bacterial or plant cultures specifically for producing PHAs. PHAs can also be recovered from open mixed cultures of biomass that are produced in biological waste treatment processes serving needs of environmental protection and residuals management.

PHAs are polyesters that can be utilized as substitute polymers in modern day plastics that are based currently on, for example, polypropylene, polystyrene, and polyethylene. PHAs have further utility in specialized applications distinct from conventional fossil fuel based polymers due. in part, to the fact that they are completely biodegradable down to carbon dioxide and water and they are generally biologically compatible materials. PHA based plastics are, therefore, bioplastics that can be both biobased and biodegradable. Within the family of PHAs, a wide range of mechanical properties is possible, ranging from stiff elastic to more rubbery. Thus, they may exhibit engineering material properties similar to many non-biobased, non-degradable polymers ubiquitously in use commercially today.

PHAs may be developed and extracted from raw material containing PHA-rich-biomass. These PHA-rich-biomass batches may be delivered to, for example, a refinary from any number of sources. Common sources include biological wastewater treatment plants where organic carbon residuals found in water and sludges can be converted by the surplus biomass to yield to a PHA-rich-biomass. Such batches of PHA-rich-biomass often contain at least 35%, but preferably more than 40%, and most preferably more than 50% of dry weight as homo-polymers, co-polymers, or copolymer blends of polyhydroxyalkanoates. One challenge faced by refineries is to accommodate batch-to-batch variability of the delivered PHA-rich-biomass.

Without limitation, said batch-to-batch variability may be with respect to the PHA-content in the biomass, the thermal stability of the PHA-in-biomass, the type of PHA, the average molecular mass of the PHA-in-biomass, and the non-PHA biomass quality. Thus, biomass should be prepared for a PHA extraction process. For example, in one embodiment the biomass may be prepared such that the thermal stability of the PHA-in-biomass is high as measured by a thermal decomposition temperature of greater than 270 °C. In other embodiments, preparation may include achieving standards in dryness of the biomass and providing dried biomass with a particle size that is both compatible with the methods and processes of extraction, as well as the needs and interests of the best possible productivity for the recovery process.

Given that batch-to-batch quality of the PHA-rich-biomass delivered to a centralized refinery will vary, there is a need to enable a centralized refinery to accommodate a wide degree of input raw material quality variability while producing a PHA as a product with quality defined within narrow well-controlled boundaries. Such a need is not easily addressed, as it involves problems regarding the assessment of PHA-in-biomass and tuning the batch-to-batch specific PHA recovery conditions in order to deliver a product of consistent quality in molecular mass while maintaining the maximum possible PHA-rich-biomass loading to the batch process for good gel formation and overall best available process productivity and economy.

Commonly reported hurdles that have prevented PHAs from wide spread utilization are the costs of production due either to the costs of refined feedstocks for the PHA-rich biomass production, energy of sterilization for pure culture systems, or else the extraction and recovery of purified PHA from biomass. Feedstock cost reduction has been shown to be feasible through the use of organic residuals found in wastewater and sludge, Sterilization costs have been shown to be avoidable through the art of applying ecological selection pressures in open mixed culture processes. Notwithstanding these upstream approaches to reduce PHA production costs, the downstream burden of cost for extraction and recovery remains a reported on-going challenge.

Additional hurdles arise with regard to extracting PHAs from biomass. The most readily applied method for extracting PHAs from biomass is by using one or more solvents and more specifically chlorinated solvents. However, if one is to avoid the use of chlorinated solvents, then so-called PHA-poor solvents may be employed. PHA-poor solvents may be defined as a solvent or solvent mixture. These PHA-poor solvents will begin to extract PHA from biomass to form a PHA-rich solution when the PHA-rich biomass and solvent are mixed and heated to at least an empirically definable temperature threshold that may or may not be above the solvent boiling point.

Generally, the range of temperatures for extracting PHA with PHA-poor solvents is between 100 and 160 °C. Higher temperatures are generally more appropriate for dissolving more crystalline PHAs. Degree of crystallinity may be influenced by the handling history of the PHA-in-biomass. Potential extent of crystallization may also be influenced by the type of copolymer including, for example, the commonly produced kinds of PHA including poly(3-hydroxybutyrate) (PHB) and copolymer blends of 3-hydroxybutyrate with 3-hydroxyvalerate (PHBV).

One challenge with PHA extraction at elevated temperatures is to avoid, or at least to minimize, the extent of the polymer degradation that takes place during the recovery process. WO2012/022998A1 describes a method or process whereby the stability of PHA-in-biomass is enhanced in order to permit for PHA recovery with the outcome of reduced molecular weight degradation kinetics at elevated extraction temperatures. However, improving the stability of the polymer in the PHA-rich biomass by methods of WO 2012/022998A1 may not impede all forms of chemical reactions that may ensue during an extraction process at elevated temperatures. Degradation of the polymer may anyway occur in solution (albeit to a lesser extent) with rates generally depending on reactant concentrations and temperature. Both the amounts of polymer dissolved in solvent and dissolved non-PHA biomass constituents may contribute to the pool of reactants promoting polymer chain scission reactions.

Further, for some PHA-poor solvents, after PHAs are extracted into solution, the polymers tend to form a loose crystalline network and thereby form a physical gel as the PHA-rich solvent solution is cooled down from the extraction temperatures. Tendency for formation of physical gels with PHA-poor solvents is recognized (US patent 6,087,471). Due to the historic experience of low thermal stability of PHAs, such gels have been considered in processes to form PHA into articles (US patent 4,360,488) whereby higher processing temperatures may be avoided. Notwithstanding, physical gel formation has been considered a general disadvantage to methods or processes of PHA extraction from biomass. US patent 7,226,765 discloses a solvent extraction process, for extracting PHA from bacteria and plants, that avoids or minimizes gel formation. US patent 6,087,471 further describes a PHA solvent extraction process at high temperature and pressure, yet this process is optimized to avoid reported undesirable gel formation. As an outcome, the predominant method in the art has been described as a solvent extraction process wherein a PHA-rich solution is cooled and methods are applied to form a precipitate and generally avoid formation of a PHA-rich gel. Also WO 96/06179 A1 discloses a method of recovering PHA from a biomass.

Current precipitation methods, however, are encumbered with various problems. For example, one method used to achieve a precipitation is to add a non-solvent to the PHA-rich solution. Such practice may complicate solvent recycling and recovery. A second reported method, designed to form a precipitate rather than a physical gel, is to select for conditions of more dilute PHA-rich solutions where gel networks may not form as readily or extensively. Such practice increases solvent consumption and reduces the product recovery volumetric productivity, Thus, despite reported enhanced processes, improvements to methods for extracting PMAs from biomass remain desired.

The challenges that are specific to industrial scale recovery of PHA that is produced within a biomass that is comprised of a mixed culture have not previously been reported. No such industrial scale facilities for PHA recovery from mixed cultures exist today. Activated sludge with capacity for PHA accumulation and produced as part of biological treatment of process and wastewaters is one example of such mixed cultures. A refinery receiving batches of PHA-rich activated sludge from different wastewater treatment facilities may have source dependent characteristics influenced by such things as bioprocess operating conditions (such as sludge retention time), wastewater quality, and feedstocks used for accumulating a PHA within the biomass.

Those involved in the trade of using PHA in commercial applications generally complain about batch-to-batch variability of the properties of the polymer resin. It is common that different batches of similarly sourced polymer resin as raw material may differ significantly, for example, in average molecular weight. Consistent product quality refers principally, but not exclusively, to a controlled recovered product average molecular mass and purity. There is further a need for improvements to control for variability of product quality in general. Furthermore, the specific challenge of adsorbing batch-to-batch variability of incoming material to a PHA recovery refinery handling PHA-rich mixed culture biomass is therefore understood to be ever so more essential towards achieving a viable commercial operation balancing needs in the PHA-recovery economy with the demands of reliable product quality assurance.

SUMMARY

This disclosure herein relates to methods for recovering PHA from a mixed culture biomass according to claims 1 and 15. In the method according to claim 15, a granulated PHA-rich biomass is prepared and admixed with a PHA-poor solvent. The PHA is dissolved in a PHA-poor solvent, allowing the PHA to be extracted from the PHA-containing biomass to produce a PHA-rich solvent. During this extraction, the temperature is maintained, on average, between $T_{L15}$ and $T_{U15}$ for at least fifteen minutes, but preferably less than 1 hour and more preferably less than two hours. The PHA-rich solvent is separated from residual biomass after extraction. Such separation may occur while maintaining the temperature and/or sheer stresses of the PHA-rich solvent in order to prevent gelation. The PHA-rich solvent may be transferred to a location of gelation. During transfer, the temperatures and/or sheer stresses are maintained to prevent gelation. The PHA-rich solvent is the promoted to gel. Such promotion may occur by cooling the PHA-rich solvent to a temperature at or below the gelation temperature. The solvent is then pressed away from the PHA-rich solvent gel.

In the method according to claim 1, biomass is directed into a reactor and mixed with a solvent. The solvent, with biomass in the reactor, is heated. PHA is extracted from the PHA-containing biomass by dissolving the PHA in the solvent. This forms a PHA-rich solvent. The PHA-rich solvent is transferred from the reactor through a conduit to a separator location. In the first section of the conduit, the PHA-rich solvent is maintained at a temperature that prevents gelation. In the second section of the conduit, the PHA-rich solvent is cooled to form a PHA-rich solvent gel. At the PHA separator location, the PHA-rich solvent gel is mechanically pressed to remove the solvent from the PHA-rich solvent gel.

In some embodiments, the biomass may be granulated to be with an average particle size in the range from 0.1 mm to 4 mm (Example 4). This particle size range may allow for a balance between maintaining an easier solvent-biomass separation while still managing to have good extraction efficiency with reasonable extraction kinetics.

In some embodiments, chemical stability of the PHA-in-biomass may be augmented, if needed, by a pre-extraction step (Example 7) whereby the biomass is extracted with a PHA-poor solvent but at temperatures below a lower PHA-extraction temperature limit ($T_L$ - Example 3). This lower PHA-extraction temperature is generally solvent, PHA-type, and biomass dependent and may be assessed in practice by methods such as the one disclosed herein (Example 3). We have defined a $T_{L15}$ as the temperature where 15 minutes of isothermal extraction yields a negligible mass of polymer dissolved in solution. Pre-extraction solvent is separated from the biomass and the biomass is disposed again to a PHA-poor solvent whereby, the PHA from the PHA-containing biomass is dissolved into solution at temperatures above $T_{L15}$. With analogy to $T_L$ (Example 3), we also define, by example, an upper extraction temperature ($T_{U15}$) as the temperature for 15 minutes of isothermal extraction that brings essentially all the PHA-in-biomass into solution. The temperature ranges may differ from embodiments due to numerous factors. For example we found that, for some embodiments with PHB-rich biomass, an extraction temperature range may be from $T_{L15}$ to $T_{U15}$ of 119 to 150 ºC. For a different biomass produced at pilot scale and containing a co-polymer blend of 3-hydroxybutyrate with 3-hydroxyvalerate (PHBV), and with a granulate size range between 0.71 and 2 mm, a temperature range of $T_{L15}$ to $T_{U15}$ may be between 78 to 195 °C. In yet another example using the same pilot system, a PHBV-rich biomass was produced, granulated to particle size between 0.71 and 2 mm, and contained to a greater extent copolymer blends with 3-hydroxyvalerate, which had a temperature range of $T_{L15}$ to $T_{U15}$ of 63 to 175 °C. With batch-to-batch variation, the extraction conditions of time and/or temperature for the PHA recovery may be altered systematically, following methods and embodiments presented herein.

In some embodiments, extraction times are kept to a minimum, wherein the amount of time for dissolving the PHA into the PHA-poor solvent to produce a PHA-rich solution is determined by predicting but more preferably directly or indirectly measuring PHA concentration in the solvent during the extracting step using one or more online monitoring techniques, such as those described herein (Example 10). The extraction times are estimated in the first instance for any given temperature cycle by means of predictive modelling (Example 3). In some embodiments, theoretical models based on laboratory derived kinetic constants may be refined and corrected to be more quantitatively representative in practice based on case-to-case experience with respective implementations of scaled-up installations (Examples 8 and 9). As used herein, more representative refers to model corrections that account for scaled up process mass transfer efficiencies which may not be as good as those achieved at laboratory scale.

In some embodiments, the extraction vessel may be generally closed so as to avoid boiling while producing a PHA-rich solution for PHA-poor solvents with boiling points less than the extraction temperature. The PHA-rich solution may be separated from the biomass, disposing the PHA-rich solution out of the system preferably by maintaining conditions of temperature and/or mixing shear stress so as to maintain a solution without onset of gelation (Example 11 ). By cooling with or without mixing, gelation of the exited PHA-rich solution may be strategically placed, and the PHA may be readily recovered from the resultant gel by mechanically engaging the gel and thereby forcing solvent exudation.

The degree of solvent loading with PHA-rich biomass may influence the molecular weight degradation rate in some embodiments. This degradation rate may be expressed on the basis of average number of polymer chain scission reactions that ensue during extraction (Example 8). Given information of the PHA-in-biomass molecular weight (Example 5) and specific demands in criteria for the extracted polymer quality, one may establish the operating parameters that do not exceed a maximum number of "allowable" scissions during extractions. For example, the solvent loading with biomass to the extraction process is an important operating parameter in some embodiments. This constraint is with respect to the goal to recover a PHA meeting a quality criterion of product molecular weight. Thus, while the PHA-type and characteristics of the biomass may influence the operating conditions of time and temperature for the PHA extraction, the PHA-in-biomass molecular weight, and the chemical stability of the PHA with the biomass during extraction may determine the maximum PHA solvent loading that can be applied for a particular PHA-rich biomass batch, Generally, solvent loadings of less 100 g-PHA/L are anticipated for these kinds of extraction processes.

In some embodiments, additives to the process may be incorporated at various stages of the process (Example 6), with objectives to enable higher extraction loadings or otherwise manipulate the recovered polymer properties. For example, pre-extraction of the solvent soluble non-PHA biomass may be employed to permit for greater loading of biomass in the solvent for the subsequent PHA-extraction step due to a reduced resulting scission rate of polymer degradation (Example 7). An increased loading of biomass for the extraction may, in turn, allow for increased extraction volumetric productivity. Higher PHA-rich solution concentrations of higher molecular weight may promote improved gel characteristics. The improved gel characteristics may provide for a greater extent of solvent exudation from the gel. Greater solvent exudation may produce a polymer of increased purity and with a greater extent of solvent recovery. All these elements may be used in part and in combination in various embodiments to improve the robustness of the PHA recovery, the process economy, and the product quality control.

Embodiments of the methods according to the invention described herein permit for controlling a-priori the molecular weight of the polymer extracted and for real time fine-tuning the extraction time from batch to batch. The methods according to the invention disclosed herein may be applied in practice to help ensure reliable product quality and manage the process operating economy. In combination, the inventors have established, with the methods according to the invention, process that will tend to reduce the overall solvent consumption while avoiding, or at least reducing, the need and costs for post purification of the recovered polymer. Maintaining stringent criteria of polymer quality, while minimizing process steps and optimizing for the solvent use and operating economy, contribute overall towards a more economically tuned process and methods of robust and consistent PHA recovery. Such are the current demands of the industry.

BRIEF DESCRIPTION OF THE DRAWINGS

Figure 1. Progress of PHB powder dissolving in Butanol at 132 ºC based on measured changes of optical density and colour.

Figure 2. Modeled first order isothermal extraction kinetics as a function of temperature for a PHB-rich-biomass.

Figure 3. Experimental results for 15-minute isothermal extraction from a PHB-rich-biomass with model sigmoid fit with extrapolated line from $T_H$ to operational limiting temperatures of $T_{L15}$ and $T_{U15}$.

Figure 4. Empirically estimated first order isothermal extraction rate coefficient as a function of temperature and the modeled values (line) based on data of $f_{15}$ as a function of temperature.

Figure 5. Experimental results for 45-minute isothermal extraction ($f_{45}$) from a PHBV-rich-biomass with model sigmoid fit and data translated to operationally defined 15-minute extraction ($f_{15}$) with limiting temperatures of $T_{L15}$ and $T_{U15}$

Figure 6. With reference to Figure 5, model versus experimental extraction yields from a PHBV- rich-biomass with average temperature and extraction times ($T > T_{L15}$) as indicated.

Figure 7. Influence of particle size on extraction of PHA-rich-biomass.

Figure 8. Particle size distribution of a PHA-rich biomass after roll-mill crushing showing the results for the first milling pass of the biomass and the distribution of selected sub-fractions.

Figure 9. Particle size distribution of a PHA-rich biomass after roll-mill crushing showing the estimated distribution passing a 2.00 mm mesh and being retained by a 0.71 mm mesh.

Figure 10. Average scission rate for isothermal extractions (125 ºC) of a PHB-rich-biomass in 2-butanol at constant biomass loading.

Figure 11. Average scission rate as function of average extraction temperature for a PHBV-rich-biomass in 2-butanol at constant PHA-loading of 50 g/L. The time $t_{50}$ is the estimated extraction time resulting in a 50% loss of average molecular mass.

Figure 12. Allowable extraction time based on a PHA-loading of 50 g/L for a fixed 35 % loss of molecular mass ($t_{35}$) influences the expected yield for the extraction (f)

Figure 13. Influence on extraction scission rate of a 90 °C pre-wash with 2-butanol for constant loading of a PHB-rich-biomass.

Figure 14. Influence of PHA loading of PHA-rich-biomass on scissions for a given extraction time, temperature and solvent. A given biomass exhibits a trend of increased (◆) scissions with loading (). However, in general different biomass batches (□) exhibit wide variation of the polymer chemical stability during extraction.

Figure 15. An illustration of PLS Model results (with model building experimental data (○) and model validation data (■)) relating the measured average specific scission rate to the predicted average specific scission rate based on an FTIR "fingerprint" of the biomass quality for distinct biomass batches with varying PHB-rich-biomass and loading conditions but with constant solvent type, average extraction temperature, and extraction time.

Figure 16. Example of PLS chemometric model application towards maintaining optimized extraction operating conditions for consistent process productivity and product quality levels with respect to incoming batch-to-batch varying PHA-rich-biomass quality.

Figure 17. A centralized refinery receiving batches of PHA-rich-biomass (B1..Bn of variable quality) from any number of wastewater treatment plants (WWTP1...WWTPn) and producing grades of biopolymer products (PHA1... PHAn) of consistent quality alongside other recovered value added chemicals for any number of commercial markets (Customer 1... Customer n).

Figure 18. Application of the process and methods towards tuning the recovery process, and recovering a PHA of defined quality while applying the most aggressive process volumetric loading possible for overall improved economy and productivity. In general a high PHA-in-biomass average molecular mass allows for higher extraction loading with all other things being equal.

Figure 19. Flow of material and information as part of applying methods of tuning process conditions in the conversion of a variable batch-to-batch quality of PHA-rich-biomass to a PHA-rich-solvent gel with consistently controlled quality.

Figure 20. Illustration of correlation between the relative change in measured solvent viscosity dependent recirculation flow (10 L process - Example 1) and the estimated modeled PHA extraction yield (Example 3).

Figure 21. Illustration of a correlation based on a PLS model between the relative change in measured solvent viscosity dependent recirculation flow (10 L process - Example 1) and the predicted mass flow increase derived from spectra from online monitoring of the solvent by Near Infrared Spectroscopy (NIR).

Figure 22. An illustration of the isothermal (102 ºC) kinetics of PHA-rich-solvent gel formation kinetics based on increase of solvent optical density with gelation.

Figure 23. An illustration of the influence of temperature on isothermal gelation time based on the onset of gelation illustrated in Figure 22.

Figure 24. An illustration of the influence of PHA-rich-solvent concentration and PHA composition (-,♦-PHB, ▲ and ■ PHBV co-polymers) on gelation temperatures.

Figure 25. An illustration of onset of gelation with the influence of high (♦ - onset below 78 °C) and low (■ - onset by 87 °C) mixing energies given similar cooling rates (≈ - 1 .6 °C/min). Note that cooling rate was influenced by the exothermic nature of gelation.

Figure 26. An illustration of onset of gelation as determined by regression analysis of the optical density as a function of temperature for a PHA-rich solvent.

Figure 27. An illustration the available time before onset of gelation for a given PHA-rich solvent cooling curve with reference Figure 26.

Figure 28. Influence of drainage rate at constant pressure (16 bar) on solvent expression and increase of gel dry solids content from a PHA-rich-gel.

Figure 29. Influence of time and pressure on solvent expression from a PHA-rich-gel.

Figure 30. A schematic illustration of elements of the extraction process.

Figure 31. A schematic illustration of the material flow in the extraction process.

## DETAILED DESCRIPTION

This disclosure provides methods for recovering PHAs from biomass according to the claims. PHA-rich biomass may be obtained from, for example, a biological wastewater treatment process.

In some embodiments, the biomass may be conditioned for improved PHA chemical stability prior to extracting the PHAs from the biomass. Pre-treatment of the PHA-rich-biomass (as discussed further below and illustrated in Example 7) may be applied in some embodiments as a means to enable higher mass loading to the batch process with benefit of greater volumetric productivity. Therefore, the recovery process operating conditions may entail selection of pre-treatment methods when appropriate in order to maintain the process economy while still consistently delivering products within defined quality criteria of purity and average molecular mass. For example, non-PHA solvent soluble compounds can be removed from the biomass by admixing solvent and the biomass at temperatures below a PHA-poor solvent critical temperature and by removing the solvent containing these impurities from the biomass prior to PHA extraction. In another embodiment, the mixed culture biomass is accumulated with PHA, thermally stabilized, and dried prior to utilizing the PHA recovery methods discussed herein.

As used herein, the term "dried" or "drying" means the removal of water, whether partially or completely. For example, the PHA-rich biomass coming from the biological treatment process can contain at least 35%, but preferably greater than 50% w/w PHA with respect to dried biomass solids, and further the dried biomass solids can contain less than 10%, and preferably less than 2% w/w water with respect to biomass.

Solvent extraction processes for recovering PHA from plants and microorganisms are well known in the art. For example, U.S. Patent No. 6,087,471 discloses a method of recovering PHA from biomass using non-halogenated PHA-poor solvents. These and other methods provide the eventual recovery of PHAs through establishing conditions to encourage PHA precipitation from PHA-rich solutions after extraction. Precipitation is with contrast to gelation, as these disclosed precipitation methods aim to avoid PHA-rich solutions from forming stable PHA-solvent gels as the gel formation is described as undesirable.

The inventors have surprisingly found that reported biomass-solvent separation problems due to PHA-rich gel formation may be overcome by exploiting the temperature and shear dependence of kinetics of gel formation during a PHA solvent extraction and recovery process (Example 11). If PHA is to be extracted from a biomass and further processed as a physical gel, then the challenge is to ensure the formation of a gel of superior quality. Gel quality is improved by higher PHA molecular weight and an increased concentration of polymer in the gel. As mentioned above, increased PHA-rich-biomass loading to the extraction process generally tends to increase the rate of polymer degradation during the extraction. Thus, adjusting the extraction process to improve one key aspect of gel quality tends to detract from a second key parameter of the polymer average molecular weight. In an embodiment according to the invention, a maximum permissible PHA-rich-biomass loading to the extraction process is constrained to reach a minimum permissible purified PHA product molecular weight.

Higher polymer molecular mass and concentration in the gel are more desirable for the product quality and ease of product recovery. Greater polymer concentration in the gel means a greater loading of PHA-rich-biomass to the recovery process, leading to a higher extracted PHA concentration. However, increasing the biomass loading to the reactor also means an increased rate of molecular weight loss during extraction. Too much molecular weight loss may be detrimental to the gel properties for the recovery and is detrimental to the final mechanical properties of the PHA processed into plastics. Lower recovered polymer molecular weight will also significantly reduce the product range of application end, economic value. The problem is to tune the recovery process to achieve the highest possible productivity in volumetric loading to the extraction for optimum overall process economy as well as a technical process demand for an optimum in recovery process performance (gel properties) and product quality (purity and molecular mass). This problem is solved by the methods according to claims 1 and 15.

PHA-poor solvents may be utilized in ways to improve the PHA chemical stability during extraction (Example 7). With improved chemical stability, higher concentrations of PHA can be extracted per batch while still yielding a polymer of similar or greater average molecular weight. Solvent loading conditions may be determined and extraction times kept optimally based on methods discussed in examples herein (Examples 1 , 2, 3, and 8). The preferred embodiments of the invention comprise a process of batch PHA recovery from a PHA-rich-biomass with solvents at temperatures generally above 100 °C and with the capture of the recovered PHA as a PHA-rich-solvent-gel. These embodiments ensure a consistent product quality with optimized batch-to-batch volumetric process productivity. Where the PHA-rich biomass contains a blend of copolymers, the conditions applied towards extraction with PHA-poor solvents should satisfy the temperature and extraction times appropriate for recovering the most difficult to dissolve types of copolymers in the blend. Thus, the methods according to the invention disclosed herein also relate to embodiments for establishing and applying optimum conditions for recovery a PHA from a PHA-rich biomass where the type of PHA polymer in the biomass is heterogeneous in nature.

Gels of higher polymer concentration, and with a polymer of higher molecular weight, are known to be more easily processed further and a higher molecular weight of the PHA provides for a final product of improved quality (value). Accordingly, an embodiment according to the invention, relates to improved methods for PHA recovery by using a single non-chlorinated PHA-poor solvent (or solvent mixture) for simplicity in solvent recovery, increasing PHA-rich extraction solution concentrations for improved productivity, and achieving desirable gelation characteristics for better final product recovery. The embodiments according to the invention disclosed herein have been focused concurrently on applying the methods of the process with due attention to the anticipated industrial commercial practice that must be able to accommodate a batch-to-batch variation of the PHA-rich-biomass quality. In an embodiment according to the invention, methods are applied to assess the PHA-rich-biomass quality in order to determine the optimum extraction conditions that will ensure a consistent extracted product quality.

The methods according to the invention include that conditions are established for controlled extraction of PHA from a PHA-rich mixed culture biomass. Extraction is performed using non-halogenated solvents. A maximum biomass loading to the solvent is selected based on the disclosed approach that balances the challenge of achieving high volumetric productivity and such that minimal, or at least acceptable level of, average molecular weight reduction of the PHA takes place. One preferred embodiment employs a strategy wherein process and operating conditions are selected to be favourable towards the formation of a PHA-solvent gel and such that gel formation is controlled so as to not be undesirable, but rather, advantageous to the process of the polymer recovery and final product quality.

PHA-solvent gel formation kinetics are dependent, for example, on temperature, mixing intensity, solvent selection, PHA concentration, PHA molecular weight, and the type of co-polymer or co-polymer blend being extracted (Example 11). The objective of at least one embodiment is to utilize the time delay in the onset of gelation of a PHA-rich solvent solution. After PHA-rich biomass extraction and before gelation the non-dissolved biomass residual solids may be separated from the PHA-rich solution and gel formation may be placed so as to enable a semi-continuous PHA-recovery process from a batch PHA-extraction process. In one embodiment, multiple batch extraction reactors can be used to produce PHA-rich solutions wherein the multiple batch extraction reactors provide PHA-rich solutions in step to a common PHA-recovery process wherein the gelation and/or exudation of solvent is coordinated. Multiple batch extraction reactors yield solutions that are sequentially gelled and, thereafter, exuded. Effective capture and use of waste heat and further processing the PHA-rich solutions from multiple batch extraction processes into a common final PHA recovery process results in more efficient PHA recovery and reduced overall process costs per kilogram of PHA recovered. Furthermore, gels from different extraction batches may be blended, thereby facilitating production of desirable well-mixed co-polymer formulations in a process that is well below melt temperatures and so without molecular weight degradation.

In one embodiment, the residual biomass can be separated from the PHA-rich solution by filtration in the reactor. In another embodiment, the residual biomass can be separated from the PHA-rich solution by capturing the biomass in a trap outside of the reactor.

In one preferred embodiment, the time to gelation is controlled by maintaining adequate temperatures and/or mixing intensity of the solvent after PHA extraction. Adequate, times and temperatures can be established by simple laboratory tests as disclosed by examples herein. The time to gelation is partly dependent on the PHA-rich solution concentration. As it is the method objective to maximize the PHA-loading to the extraction process, factors of PHA-loading and gelation time must be coordinated.

The PHA-loading to the process is the theoretical concentration of PHA that would be achieved if all the extractable PHA in the biomass ended up in solution. The extractable PHA may be quantified, for example, by methods based on TGA (thermal gravimetric analysis), FTIR (Fourier transform infrared spectroscopy), gas chromatography, and/or methods of standardized extraction (Example 1 and Example 3). The maximum allowable PHA-loading becomes a balance of achieving objectives of high extraction yield and a high product molecular weight within a robust PHA-solvent gel that can be exuded to produce, in the end, a polymer of at least adequate molecular weight and of, at least, adequate purity.

Adequacy in this context is product application dependent. Generally an extracted polymer of greater than 90 %, preferably greater than 95 %, and most preferably greater than 98% may be considered adequate for a wide range of applications. Similarly, a weight average molecular mass greater than 350 kDa, preferably greater than 400 kDa, more preferably greater than 600 kDa, and most preferably greater than 700 kDa may be suitable for a wide range of potential applications. The methods disclosed herein enable the selection of suitable extraction operation parameters to reach a predictable product molecular weight, and so adsorb the incoming raw material variability in the operation of a PHA recovery process to deliver a product within a well-defined quality window for a specific product application.

It is furthermore desirable that the recovered PHA is with a high thermal stability as exhibited by a thermal decomposition temperature greater than 270 °C, but preferably greater than 280 °C, and even more preferably greater than 285 °C (Example 1). Thermal stability of the PHA-in-biomass or the recovered PHA may generally be assessed by thermogravimetric analysis (TGA). The thermal stability of the recovered polymer may also be understood with respect to rheology measurements whereby the material exhibits a 1 log melt stability (180 °C) of greater than 5 minutes, and preferably greater than 15 minutes (see for example WO 2012/022998 A1).

The entwined factors that influence the selection of suitable extraction process PHA-loading conditions are as follows:

- Lower PHA-loading for improved extraction yield meaning a minimum amount PHA residual remaining in the biomass after extraction.
- Lower PHA-loading for reduced kinetics of molecular weight reduction during extraction.
- Higher PHA-loading for robust gel formation,
- Higher PHA-loading for greater process volumetric productivity.
- Both lower and higher PHA-loading for optimal gel solvent exudation properties wherein, lower PHA-loading helps due to a higher product molecular weight, and higher PHA-loading helps due to a greater PHA-solvent gel concentration.

Thus, the optimal conditions of PHA-loading are constrained in balance to be sufficiently high and low. This balance point varies depending on the source of the PHA-rich biomass and the type of co-polymer or co-polymer blend that is to be extracted. An example of a method or process for establishing optimal recovery conditions on a case-by-case basis is illustrated in Example 9.

One preferred embodiment provides that a PHA-rich solution is separated from the residual biomass with temperature maintained such that gelation does not occur until the PHA-rich solution is separated from the non-soluble biomass fraction. In one embodiment, the cooling mechanism is by heat exchange that can be used to preheat solvent in a neighbouring extraction reactor. Once gelation occurs, mechanical separation may be employed to express the solvent and recover both PHA and reusable solvent (Examples 11 and 12). Ideally, the solvent should be recovered at as high a temperature as possible in order to minimize the energy used in solvent recovery.

It has been reported that precipitation of PHA after extraction in a PHA-poor-solvent can nevertheless be achieved by applying shear forces (at low polymer concentration), adding a more polar but miscible solvent, or by rapid dilution of the PHA-rich solution with cooled extraction solvent. However, all of these embodiments, that are added to avoid a gel formation, add process complication and expense:

- Adding a more polar but miscible solvent significantly increases solvent consumption, makes product recovery more challenging, and imposes further capital and operation expenses in the energy demands for solvent recovery and reuse.
- Introducing a more polar solvent may promote co-precipitation of co-extracted solvent soluble non-PHA biomass residues resulting in generally poorer product quality.
- Precipitation based on high shear forces is generally with low PHA concentration implying poor process volumetric productivity and excessive solvent and energy consumption.

In our findings with PHA recovery, the combination of a single PHA-poor solvent recovery process, with methods to maximize the allowable PHA-loading, together with optimization of gelation characteristics contribute both to an overall good process performance and a means to pro-actively control the final product quality.

PHA-in-biomass that has been thermally stabilized (see for example WO2012/022998A1) and subsequently recovered via such extraction methods in a PHA-poor solvent and captured by controlled gelation is expected to be relatively pure (> 95% purity). Naturally, PHA recovered in this gelation manner can always be further processed downstream by utilizing thermal methods or additional solvent rinsing or blending strategies to obtain even higher purity PHA or desirable value-added compounded polymer raw materials.

Chemical additives towards the formation of formulated raw polymer materials, including but not limited to, chemical scavengers, nucleating agents, stabilizing chemicals, plasticizers, functionalizers, and fillers, may be added and mixed into the PHA-rich-solution and captured in the matrix during extraction, after extraction and biomass separation, and/or during, or even after the gelation process (Example 6). Therefore, gelation of PHA brings with it advantages in anticipation of the processing of the polymer into further improved and value added raw materials and/or bioplastics. To this end, and as a practical example, we have discovered that the compounds in the class of polycarbodiimides can induce chain extension reactions when combined with a PHA-rich-solution at elevated solvent temperatures after extraction but before gelation. Such practice may be used to augment or compensate for the material properties after PHA-poor solvent extractions.

The molecular weight of the recovered PHAs and the molecular weight distribution across a blend of PHAs are principal quality factors that influence polymer physical properties, Generally, higher molecular weights are more desirable. Reducing the PHA extraction time in the reactor minimizes the decrease in average molecular mass of the PHAs that can occur during the extraction process. Thus, keeping the extraction time to no more than it needs to be increases the ability to recover the desired higher average molecular weight PHAs from the PHA-rich solution. By predicting or, more preferably, directly monitoring the concentration of extracted PHA in the solvent, it is possible to ensure that the extraction times are kept as short as possible (Example 10). Predictions can be made by practical characterization of the biomass to be extracted (Example 3). Direct monitoring of the extraction process trend may be by solvent quality monitoring using sensing methods such as spectroscopy or viscosity of the PHA-rich solution during the extraction process (Example 10).

The requisite extraction time and the extraction yield are influenced by the particle size of the dried biomass. The extraction process is mass transport limited from the biomass (Examples 2 and 3). In general, we experience that smaller particles will improve the solvent extraction kinetics and product yield. Mass transfer kinetics generally improve with increase in interfacial surface area (small particle size). At the same time the post extraction separation of the non-dissolved biomass from the PHA-rich-solution is simplified given a larger particle size. We find (Example 4) that when biomass particles are too large the extraction does not proceed into the core of the biomass particle. Further, larger particles mean less contact surface area with reduced mass transfer kinetics, longer extraction times, and, as a consequence, increased degree of molecular weight loss. The solvent penetration depth was found to be in the order of 2 mm.

In practical experiments with a particle size distribution between 0.71 and 2 mm, whereby the dominant size fraction was 1.4 mm, we experienced a good balance between extraction yield and kinetics with the ability to readily separate the extracted biomass granulate from the PHA-rich solvent. In direct comparison and for the same extraction conditions in replicated experiments with two different biomass batches, comparing distinct granulate size distributions of 0.71 to 2 mm and of 2 to 3.15 mm, up to 23 percent loss in extraction efficiency was observed with the larger granulate distribution. Therefore, a dominant granule particle size in the order of 1 mm is considered to be optimal for industrial practice of PHA recovery from such biomass.

From practical work in grinding a dried PHA-rich biomass to become a granulated biomass with a particle size distribution between 0.71 and 2 mm, we found that a 0 5 mm filter mesh would retain most of the biomass after extraction. In the experimental example embodiment, a 0.1 mm was further utilized to trap biomass fines that were present or formed during extraction. Based on the findings of solvent penetration in the biomass, the biomass particles in the extraction system should be less than 4 mm in nominal diameter. We have found In practice that a granulated biomass with particle size distribution greater than 0.5 mm and less than 2 mm provided a good balance between performance in extraction and practical ease of PHA-rich solvent separation. By keeping the dominant granulate particle size of around 1 mm, formation or minor presence of biomass particle fines of larger than 0.1 mm could be readily screened out of the PHA-rich solvent stream after the bulk of the biomass was separated from the solvent.

Thus, a biomass granulate particle size distribution of less than 4 mm, and ideally with dominant fraction of the order of 1 mm and, fines of greater than 0.1 mm was discovered to strike a balance between contrasting optimization considerations of practical simplicity for the residual biomass separation, process efficiency in extraction time and, by association loading potential. In this manner, effective extraction times could be less than 5 hours, but preferably less than 2 hours and even more preferably less than 1 hour.

Those skilled in the art will understand that the solvent-biomass boundary layer at the interface between biomass particles and the solvent bulk volume should be theoretically as thin as possible. Since average molecular weight degradation kinetics are dependent on concentration, the locally concentrated PHA-rich solution at the particle-solvent interface should be transported and diluted into the bulk solution as quickly as possible. Therefore, mixing is preferred during extraction. Mixing of the extraction solvent with respect to the biomass in the extraction reactor can improve the extraction yield, increase the extraction kinetics, and thus mitigate polymer degradation during extraction, The limits of potential for the polymer extraction and degradation kinetics may be evaluated in standardized laboratory scale experiments (Example 3).

In practice, mixing energy is preferably applied to maintain gentle but relatively rapid dispersion and dilution of PHA extracted away from the biomass particle-solvent interface throughout the complete solvent volume without breaking up the biomass particles. Too aggressive mechanical mixing conditions have been found to reduce the biomass granules to a fine powder that readily breaks through simple filtration screens and this leads to undesirable contamination of the final product. Therefore, in one embodiment, solvent may be pumped through a filter bed in modes of fluidizing bed up flow or pressure gradient driving solvent flow through a packed biomass bed of granules, rather than mixing the granules freely within the solvent volume.

The kinetics of PHA extraction in a PHA-poor solvent are influenced by temperature. The influence of temperature for dissolving a given PHA from a given biomass in a given PHA-poor solvent can be readily determined by a simple laboratory procedure (Example 3). This procedure is applied to a finely ground biomass with aim of providing for a high contact surface area and resulting maximum kinetics of mass transfer more specifically related to a given PHA in a given biomass. The procedure can also be applied to the granulated biomass as a means to quantify the influence in practice of the particle size distribution on the resulting extraction kinetics. Such procedures indicate for a lower temperature limit ($T_L$) below which relatively negligible if any extraction of PHA will be anticipated to occur over a time scale of minutes to fractions of an hour. At the same time an upper temperature limit ($T_U$) may be defined for which significant PHA will be anticipated to become extracted on a time scale in the order of minutes to fractions of an hour. The procedure according to claims 1 and 15 provide for empirically derived kinetic constants of extraction as a function of temperature above the $T_L$ value. Based on model assumptions, of for example first order extraction kinetics, the temperature dependence of the extraction kinetics can be estimated and from these model calculations, a $T_{L15}$ and $T_{U15}$ are derived. The $T_{L15}$ is a lower temperature where negligible extraction will occur within 15 minutes, and the $T_{U15}$ is an upper temperature where significant PHA extraction will occur within 15 minutes. $T_{L15}$ and $T_{U15}$ are not the same for all PHA-biomass batches due to differences in the granulated particle size distribution, biomass characteristics, and/or differences in the type of PHA in the biomass. The present method provides for augmented definition of extraction process conditions over and above those that have been previously disclosed for mixed-culture PHA-rich biomass. The PHA extraction temperature can vary from batch to batch. Therefore, we define a PHA-recovery process with a requisite extraction temperature on average between $T_{L15}$ and $T_{U15}$. We define the extraction time and the average extraction temperature from the point where the PHA-rich biomass is exposed to PHA-poor solvent temperatures greater than $T_{L15}$.

Based on a defined dependence of extraction kinetics with temperature and knowledge of the heating time-temperature trend for the extraction reactor, the anticipated extraction progress in time is predicted (Example 3). These predictions may be used to place practical upper time limits for the extraction process. The theoretical model predictions may be tuned with practical adjustment and correction factors to reflect the performance boundaries or mass transfer limitation embodied in specific scaled up extraction facilities,

The loss of number average molecular mass of PHA during the extraction process can be reported in terms of the average number of polymer chain scissions:

$$N_s(t) = \frac{M_n(t=0)}{M_n(t)} - 1 \qquad \text{EQ-1}$$

where

$N_s$ = Chain Scission Number as a function of extraction time (t)
$M_n$ = Number average molecular mass as a function of extraction time (t)

For example, if the original number average molecular mass decreases from 800 KDa to 100 kDa over the course of a 1 -hour extraction at a given average extraction temperature, then the 1-hour scission number is 7 at that given temperature. Every polymer chain is cut on average 7 times during 1 hour at that given temperature.

If the polydispersity index is approximately constant with average loss of molecular mass during extraction, then the scission number can also be calculated instead with data of weight average molecular mass ($M_w$):

$$N_s(t) = \frac{M_n(t=0)}{M_n(t)} - 1 = \frac{M_w(t=0)}{M_w(t)} - 1, \text{ where } PDI = \frac{M_w}{M_n} \approx constant \qquad \text{EQ-2}$$

The number of scissions per unit time for a given temperature suggests an average temperature specific scission rate:

$$\bar{R}_s = \frac{1}{t} \int_0^t N_s \, dt \qquad \text{EQ-3}$$

where,

$R_s$ is the estimated average scission rate established with respect to a given temperature over a specified time period. Since a greater number of scissions would correspond to a greater amount of polymer in a given system, comparisons of scission counts between experiments may be referenced on the basis of scission density or specific scission rate. The average specific scission rate or $\bar{r}_s$ refers to the number of scissions per mass or concentration of polymer in the extraction process:

$$\bar{r}_s = \frac{\bar{R}_s}{X_p} \qquad \text{EQ-4}$$

where $X_p$ is the mass of polymer introduced to the extraction process. Without limitation, and as a pragmatic reference, we have calculated the average specific scission rate based on the theoretical maximum concentration of PHA if 100 percent of the loaded PHA mass were to be extracted. In other words, $X_p$ in Example 8 refers to the PHA-loading to the extraction process. The greater $\bar{r}_s$ is, the more the polymer molecular weight loss will be for a given loading, temperature, and extraction time. In fact we have found that it is possible to define $\bar{r}_s$ as a function of biomass loading for a given extraction temperature (Example 8) based on methods of applied chemometrics:

$$\bar{r}_s = f(X_p + X_n) \qquad \text{EQ-5}$$

where $X_n$ is the non-PHA fraction of the biomass loaded to the extraction process. Thus in a calibrated system for a given extraction temperature and solvent (Example 8), the loading dependent specific scission rate may be described. We have found that assessment of the scission rate for the polymer during the recovery process is central to accommodating batch-to-batch PHA-rich biomass quality variability with batch specific tuning of the PHA process recovery operating parameters.

With further information of the PHA-in-biomass molecular weight distribution (Example 5) and extraction time, a biomass solvent loading limit may be defined for which the recovered polymer is anticipated to stay above a defined product quality lower limit with respect to molecular weight (Example 9). The biomass loading constraint may be compared to the resultant PHA solvent loading to be applied as this relates to practical process constraints for product recovery by gelation.

Where increased PHA solvent loading is appropriate, further measures may be taken to enable an increase in overall biomass solvent loading without undue sacrifice to the final product quality. Without limitation, such measures may include pre-treatment (Example 7), post-treatment (Example 6), and/or the application of chemical additives (Example 6). As additional measures are likely to incur additional expense, it is seen to be generally commercially advantageous in the polymer recovery process economy to have means that enable to justify on a batch-to-batch basis explicitly when additional pre-treatment measures are appropriate or may be offset by greater returns due to improved recovered polymer product value. We have found methods and processes disclosed by examples herein to enable such case-to-case tuning of the polymer recovery process in order to ensure final product quality by the purposeful accommodation of observed batch-to-batch variation of the biomass quality, the polymer type, the polymer molecular weight, and the biomass polymer content.

Loading of biomass into the solvent for the extraction process introduces a mass of PHA-biomass ($X_p$) and non-PHA-biomass ($X_n$). For a given PHA-rich biomass, the objective is to load the solvent in the extraction process with as much mass of PHA as possible with the constraint of limiting the extraction chain scission number so as to deliver a product equal to or above a defined number average molecular mass. We have found that for a given biomass loading, the number of chain scissions determining the recovered product molecular weight distribution is dependent on:

- the PHA loading ($X_p$),
- the non-PHA biomass loading ($X_n$),
- type and amount of impurities associated with the non-PHA biomass loading,
- the average extraction temperature above $T_{L15}$,
- the type of PHA-poor solvent, and
- the extraction time.

In general, for a given biomass loading and constant extraction temperature, we observe a constant scission rate. In other words, the number of scissions at a constant temperature increases linearly with time:

$$N_S(t) = \frac{M_n(t=0)}{M_n(t)} - 1 = \bar{R}_S \cdot t \qquad \text{EQ-6}$$

However, we found that the influence of the non-PHA biomass loading on scission number may vary from biomass to biomass batch. The non-PHA biomass chemical content contributes to the chemical stability of the polymer during extractions. The complexity and potential for variability of the non-PHA biomass composition challenges the assessment of contributing chemical elements. Notwithstanding this complexity and variability, we have found that it is possible to calibrate the scission dependency for a given solvent and extraction temperature. Based on methods of chemometrics, the average specific scission rate for a given biomass and extraction temperature is predictable based on calibration experiments. Thus in a production scenario for a centralized facility recovering PHA from PHA-rich-biomass delivered from a variety of production sources, loading conditions for extraction may be tuned on a case by case basis in order to maintain consistent and optimal process efficiency and product quality.

In general, the degradation kinetics of PHA in PHA-poor solvents increases with concentration of non-PHA biomass loaded with the PHA-biomass in the extraction reactor. At the same time, sufficient PHA must be extracted in order to ensure the beneficial formation of a stable gel after extraction and PHA-rich solvent separation from the biomass. We found that gel formation becomes more reliable at PHA concentrations of around 30 g-PHA/L, but PHA-gel concentrations above 40 g-PHA/L are more preferred. Ease of solvent expression from the gel was generally found to be improved with higher PHA-gel concentration and higher PHA molecular weight. Higher gel concentrations can also be achieved by evaporating solvent from the gel before pressing.

An embodiment of the present invention includes determining adequate time for PHA extraction for a particular biomass and with the particular solvent used during the extraction process. As used herein, the phrase "extraction time" refers to the amount of time that the PHA-containing biomass is kept in the PHA-poor solvent at or above the $T_{L15}$ extraction temperature. This "extraction time" is the time it takes for the PHA to become dissolved in the PHA-poor solvent to produce a PHA-rich solution.

A second parameter is the "recovery time" during the process. The "recovery time" is the time available to separate the PHA-rich solution from the spent biomass solids before the onset of gelation. In order to assess the process criteria of a recovery time, a series of small-scale trials may be performed to assess gel formation kinetics for a particular combination of solvent and PHA composition at a given temperature over time. For example (Example 11), the onset of gelation of PHA-rich 2-butanol may be delayed for a period of time following the cooling of the PHA-rich 2-butanol from 125 °C to 102 °C, providing a window of time in which the PHA-rich 2-butanol can be separated from the residual biomass and removed from the extraction reactor. We also found that application of shear stress to the PHA-rich solvent can be applied to suppress the gelation point to lower more narrowly defined temperatures than without the application of shear stress.

One wishes to increase as much as possible the PHA-rich biomass loading in the solvent. In order to not go under quality criteria of recovered PHA molecular weight average, the degree of loading may be constrained by the average molecular weight of the PHA-in-biomass and the average number of scissions that are defined acceptable in the recovery process. A greater loading improves the polymer-gel concentration facilitating a gel exhibiting a higher degree of readily expressible solvent. Where loading is not sufficiently high, solvent can be evaporated from the gel before expressing solvent. At the same time an increase of concentration of PHAs in the solvent influences the kinetics of gel formation. The process control needs to accommodate non-PHA non-dissolved biomass separation before gelation starts. For example (Example 11), the effect of concentration of PHA on gelation temperature can readily be determined for a given mixture of PHAs in 2-butanol. In one embodiment of the present invention, the PHA-loading of the PHA-rich solvent is between 30 and 100 grams of PHA per liter of solvent. Increased levels of PHA in the PHA-rich solution generally reduce the recovery time, for a given temperature, to achieve solvent separation in advance of the subsequent gelation.

The temperature at which gelation is applied is preferably as high as possible while still being under the boiling point for the PHA-rich solution. Reducing the extent of solvent cooling can be used to improve the process energy efficiency for subsequent solvent recycling and recovery based on evaporation. Fresh solvent for the next extraction can be pre-warmed with the heat drawn for the purposes of controlled gelation.

One or more online monitoring techniques can be employed to control for the extraction time for obtaining a PHA-rich solution. In one embodiment, the trend of PHA extraction is determined as a function of viscosity (Example 10). The change of polymer concentration and polymer molecular weight in solvent solution influences the solvent viscosity which can be monitored, for example, by trends of mixing torque or mass flow changes in recirculation pumping during extraction. In another embodiment, the progress of PHA-rich solution formation can be monitored during the extracting step using near infrared (NIR) spectroscopy (Example 10). Measurement trends referenced to the solvent pre-extraction viscosity or NIR spectra were found to be indicative of the extraction process without need for absolute calibration of concentration dependent solvent viscosity or spectral changes. Other indirect monitoring methods such as an observed influence of the PHA extraction on the solvent vapour pressure can be applied to indicate progress of the extraction.

Solvents suitable for use in the preferred embodiments discussed herein include PHA-poor solvents or solvent mixtures. As used herein, the phrase "PHA-poor solvent" means a solvent or solvent mixture that generally exhibits negligible kinetics of dissolution below an empirically definable temperature limit ($T_{L15}$) (Example 3). In 2-Butanol, the $T_{L15}$ is nominally 119°C for PHB and lower than 100 °C for co-polymers of PHBV richer in 3HV. Biomass containing blends of co-polymers may exhibit a multiple of limiting temperatures corresponding to the respective distinct fractions of co-polymer blends present. Such blend fractions may be extracted sequentially or concomitantly in an extraction process. Where a multiple of distinct co-polymer types exist in the biomass, the extraction time and temperatures may be tuned to the PHA-type in the blend of highest $T_{L15}$.

We have found that solubility of PHA in a PHA-poor solvent may not be readily predicted by existing established theories of solubility. One may speculate that these solvents allow the polymer to effectively melt in solution at temperatures lower than the polymer melting point by acting in some way as plasticizers that weaken polymer chain interactions. Notwithstanding the possibility for other theories or the future development of theoretical predictive models, the suitability of a solvent or solvent mixture to be a PHA-poor solvent may be readily evaluated empirically. Based on our own practical experience and the literature, PHA-poor solvents are generally anticipated to be found within the groups consisting of aliphatic alcohols, ketones, esters, and/or aromatic hydrocarbons.

While we have found that solubility theory does not adequately predict the suitability of a solvent to be a good PHA-poor solvent, we have successfully applied simple practical testing methods at test tube scale to screen suitability of solvents, or mixtures, as PHA-poor solvents.

Based on practical experience and literature, one may anticipate that PHA-poor solvents are to be found within the groups consisting of aliphatic alcohols, ketones, esters, and/or aromatic hydrocarbons.

In another embodiment, the solvent dissolves non-PHA components of the PHA-rich biomass such that upon cooling, the PHA-rich solution forms a gel and the non-PHA components of the biomass remain in solution. Thus, dissolved non-PHA biomass components can be separated with the solvent from the extracted gelled PHA. Desirable PHA-poor solvents are those that are capable of dissolving at least 30 grams PHB per liter of solvent at temperatures in the range of about 100°C to about 160 °C, and further form a solvent-gel when cooled to temperatures preferably above 60 °C. A growing list of known "PHA-poor solvents" are reported in the literature, including but not limited to ketones such as acetone, methyl ethyl ketone (MEK) and methyl isobutyl ketone (MIBK), alcohols such as propanol, butanol, and pentanol and isomers thereof, toluene, and propylene carbonate. Gelation characteristics of PHA-poor solvents are less well-reported but butanol and toluene are two good examples. Combinations of one or more of the above mentioned preferred embodiments of the invention are also contemplated and are encompassed by the present disclosure. Such combinations are readily apparent to those skilled in the relevant art. Further, the following examples are merely illustrative and should not be considered as limiting the scope of the claims in any way, as the examples and other equivalents thereof will become apparent to those versed in the art Therefore, one embodiment relates to a method for recovering PHA from a mixed culture biomass. A granulated, PHA-rich biomass is prepared. The PHA-rich biomass is admixed with a PHA-poor solvent, The PHA is extracted from the PHA-rich biomass to produce a PHA-rich solvent. This extraction occurs by dissolving the PHA in a PHA-poor solvent at temperatures above a limiting extraction temperature ($T_{L15}$). The temperature of the PHA-poor solvent is maintained during extraction so that it is between $T_{L15}$ and $T_{U15}$. The maintained temperature and extraction occurs for a period of time greater than 15 minutes, and preferably less than 1 hour but most preferably less than 2 hours. After the PHA is extracted from the PHA-rich biomass, the PHA-rich solvent is separated from the residual biomass. During this separation, temperature is maintained so that it remains above a gelation temperature. The PHA-rich solvent is transferred to a location of gelation During transfer, the temperature and/or sheer stresses of the PHA-rich solvent are maintained in a manner sufficient to prevent gelation. When the PHA-rich solvent is at a predetermined location, it is cooled to a temperature at or below the gelation temperature to promote PHA-rich solvent gelation. Such cooling may occur with or without mixing. Solvent is then pressed away from the PHA-rich solvent gel.

In some embodiments, the biomass granulate is prepared so that it has a particle size distribution nominally between 0.1 and 4 mm. In a preferred embodiment, the biomass granulate is prepared so that it has a particle size distribution with a dominant fraction between 0.5 and 2 mm.

In some embodiments, the PHA is recovered in a system. These methods comprise additional steps, so that a biomass loading to the system is selected based on a chemometric model calibration such that the recovered PHA weight average molecular mass is at least 350 kDa. In a preferred embodiment, the recovered PHA weight average molecular mass is greater than 500 kDa. In another preferred embodiment, the recovered PHA weight average molecular mass is greater than 700 kDa.

In methods utilizing an extraction system, the methods may include selecting a biomass loading to the extraction system based on a bench scale trial to determine the specific scission rate, so that the recovered PHA weight average molecular mass is at least 350 kDa. In a preferred embodiment of these methods, the recovered PHA weight average molecular mass is greater than 500 kDa. In a more preferred embodiment, the PHA weight average molecular mass is greater than 700 kDa.

In some embodiments, the PHA is recovered in a reactor. Such embodiments include a biomass loading to the reactor to achieve a minimum PHA-rich solution concentration of greater than 20 g-PHA/L In a preferred embodiment, the minimum PHA-rich solution concentration is greater than 30 g-PHA/L. In a more preferred embodiment, the PHA-rich solution concentration is greater than 40 g-PHA/L.

In some embodiments of the methods discussed herein, pressing the solvent away from the PHA-rich solvent gel produces PHA having a purity greater than 90%. In preferred embodiments, pressing the solvent away from the PHA-rich solvent gel produces PHA having a purity greater than 95%. In more preferred embodiments, pressing the solvent away from the PHA-rich solvent gel produces PHA having a purity greater than 98%.

In some embodiments, the mixed cultured biomass is produced in a biological wastewater or process water treatment process.

In some embodiments, the PHA-in-biomass is thermally stable with a PHA-in-biomass thermal decomposition temperature greater than 270 °C. In preferred embodiments, the PHA- in-biomass thermal decomposition temperature is greater than 280 °C.

In some embodiments, the PHA-containing biomass is dried. The water content of the dried PHA-containing biomass is less than 10%. In a preferred embodiment, the water content of the dried PHA-containing biomass is less than 2 % w/w water/biomass. In some embodiments, the dried PHA-containing biomass is at least 35% w/w PHA/dried biomass. The dried PHA-containing biomass is at least 50% w/w PHA/dried biomass in preferred embodiments.

In some embodiments, the biomass is subjected to a pre-extraction process with a solvent where the solvent is maintained at a temperature below $T_{L15}$.

In some embodiments, chemical additives including molecular weight stabilizers and/or polymer compounding agents, such as chemical scavengers, antioxidants, nucleating agents, plasticizers and/or reactive polymer modifying agents, are added to the PHA-poor solvent before or after the formation of a PHA-rich solvent,

In some embodiments, chemical additives including molecular weight stabilizers and/or polymer compounding agents, such as chemical scavengers, antioxidants, nucleating agents, plasticizers and/or reactive polymer modifying agents, are added and blended into the gelated mass before and/or during mechanically engaging the gel to reduce the solvent content.

In some embodiments, more than one batch of gelated PHA rich solvent are blended into a combined gelated mass before and/or during mechanically engaging the combined gels to reduce the solvent content.

The biomass may be separated from the PHA-rich solvent. In some embodiments, the biomass is separated from the PHA-rich solvent by filtration in an extraction reactor. In other embodiments, the biomass is separated from the PHA-rich solvent by capturing at least a fraction of the biomass in a trap outside of an extraction reactor.

In some embodiments, the gel pressing is achieved by mechanically engaging the gel with pressures of between 5 and 30 bar.

Some embodiments may further include circulating the solvent through the biomass in an extraction reactor. During the process of circulating the solvent through the biomass in the extraction reactor, PHA in the biomass is dissolved into the circulating solvent.

Some embodiments may further include circulating the biomass in the solvent in an extraction reactor. During the process of circulating the biomass in the solvent in the extraction reactor, PHA in the biomass is dissolved into the solvent.

In some embodiments, more than one batch extraction reactor is serviced by a common location of PHA-rich solvent gel.

In some embodiments, the PHA-poor solvent is one or more solvents from the groups consisting of aliphatic alcohols, ketones, esters, and/or aromatic hydrocarbons.

In some embodiments, non-dissolved biomass is separated from the PHA-rich solvent. This biomass is subjected to an incineration or pyrolysis process in some further embodiments, phosphorus is recovered from the incineration or pyrolysis process.

In some embodiments, other chemical products such as lipids and fatty acids are recovered as part of the solvent recovery after extraction and separation of the solvent from the PHA-rich solvent gel.

The disclosures herein relate to another embodiment for a method of recovering polyhydroxyalkanoates (PHA) from PHA-containing biomass. Biomass is directed into a reactor. In the reactor, a solvent is mixed with the biomass. The solvent and biomass are heated in the reactor. PHA from the PHA-containing biomass is extracted. The extraction occurs by dissolving the PHA in the solvent to form a PHA-rich solvent. The PHA rich solvent is transferred from the reactor to a PHA separator where the PHA is separated from the PHA-rich solvent. The PHA-rich solvent is maintained at a temperature that prevents the PHA-rich solvent from assuming a gelation state while the PHA-rich solvent is transferred to the PHA separator. The PHA-rich solvent is then cooled to form a PHA-rich solvent gel. The PHA-rich solvent gel is mechanically pressed at the PHA separator location, causing the solvent to be removed from the PHA-rich solvent gel.

In some embodiments, the solvent mixed with the biomass is a PHA-poor solvent.

In some embodiments, the biomass and solvent are heated in the reactor to a temperature above a limiting extraction temperature ($T_{L15}$) during the extraction of PHA from the PHA-containing biomass.

In some embodiments, the temperature of the solvent in the reactor is maintained, on average, at a temperature between $T_{L15}$ and an upper temperature ($T_{U15}$) for a period of time greater than 15 minutes during the process of extracting PHA maintaining.

In some embodiments, the biomass is ground to form biomass granules before being directed into the reactor. The granules are then directed into the reactor and contacted with the solvent.

In some embodiments, the biomass is formed into granules. The granules have a particle-size distribution normally between 0.1 and 4 mm.

Some embodiments produce a PHA-rich solvent having a PHA concentration of at least 20 g-PHA/L.

In some embodiments, mechanically pressing the PHA solvent gel produces PHA having purity greater than 90%.

In some embodiments, the PHA in the biomass directed to the reactor is thermally stable. The PHA in the biomass includes a thermal decomposition temperature greater than 270°C.

In some embodiments, PHA-rich solvent is separated from non-dissolved biomass. The temperature of the PHA solvent is maintained sufficient to prevent gelation during this separation process.

In some embodiments, the PHA-rich solvent is directed through a heat exchanger disposed in a first section of a conduit. The PHA-rich solvent is heated by transferring heat from the heat exchanger to the PHA-rich solvent. In further embodiments, the PHA-rich solvent is directed through a heat exchanger in a second section of the conduit and then cooled by transferring heat between the heat exchanger in the second section of the conduit and the PHA-rich solvent.

In some embodiments, the PHA-rich solvent gel is mechanically pressed to separate PHA from the PHA-rich solvent gel and form a PHA cake.

In some embodiments, the PHA cake is directed to a dryer. The PHA cake is then dried.

In some embodiments, the PHA-rich solvent gel is mechanically pressed to expel spent solvent from the PHA-rich solvent gel. The spent solvent is purified by subjecting the spent solvent to an evaporation process. A solvent vapour is produced. The solvent vapour is then condensed to form a purified solvent.

In some embodiments, chemical products such as lipids and fatty acids are recovered as part of the purification and recovery of the extraction solvent,

In some embodiments, the biomass is separated from the PHA-rich solvent. The separated biomass is subjected to an incineration or pyrolysis process. In a further embodiment, phosphorus is recovered from the residual material after incineration or pyrolysis.

In some embodiments, the solvent is circulated through the biomass in the reactor. During the process of circulating the solvent through the biomass in the reactor, PHA in the biomass is dissolved into the circulating solvent.

In some embodiments, the PHA-poor solvent is one or more of the solvents from the groups consisting of aliphatic alcohols, ketones, esters, and/or aromatic hydrocarbons.

In some embodiments, the biomass loading to the reactor is adjusted such that the PHA loading is between 20-100 g-PHA/L.

In some embodiments, the biomass is formed into granules with the granules having a particle size distribution normally between 0.1 and 4 mm prior to introducing the biomass into the reactor. The biomass is then mixed with a PHA-poor solvent in the reactor. The PHA in the biomass directed to the reactor is thermally stable and includes a thermal decomposition temperature greater than 270°C. After extracting PHA from the PHA-containing biomass, the PHA-rich solvent is separated from the biomass. During the separation process, the temperature of the PHA solvent is maintained sufficient to prevent gelation.

In some embodiments, an online monitoring process for monitoring kinetics of PHA extraction is provided during the step of extracting PHA from the PHA-rich biomass. In further embodiments, the online monitoring process comprises measuring changes in the viscosity of the PHA-poor solvent over a selected time period. Such online monitoring process may further include, in additional embodiments, measuring temperature compensated changes in the viscosity of the PHA-poor solvent over a selected time period. In yet other embodiments, the online monitoring process comprises monitoring the PHA-poor solvent by near infrared spectroscopy (NIR). In some embodiments, the online monitoring process comprises monitoring the trends of pressure as a function of temperature in an extraction vessel.

In some embodiments, the step of extracting PHA from the PHA-containing biomass includes providing an online monitoring process for monitoring kinetics and progress of PHA extraction. In some embodiments, the online monitoring process comprises measuring changes in the viscosity of the PHA-poor solvent over a selected time period. In some embodiments, measuring the changes in viscosity includes measuring temperature compensated changes in the viscosity of the PHA-poor solvent over a selected time period. In some embodiments, the online monitoring process comprises monitoring the PHA-poor solvent by near infrared spectroscopy (NIR). In other embodiments the online monitoring process comprises monitoring the trends of pressure as a function of temperature in the reactor.

### Example 1 - Common Analytical Methods and Materials

### Thermal Gravimetric Analysis (TGA)

Finely ground dried PHA-rich-biomass samples of between 2 and 10 mg were weighed and heated in inert nitrogen atmosphere from room temperature to 550 °C. The sample temperature was raised to 105 °C at a rate of 10 °C and the weight was allowed to equilibrate at 105 °C for 10 minutes. Moisture loss from the sample was assessed at 105 °C. The temperature was again raised at a rate of 10 °C and weight loss was recorded up to 550 °C. Ash content of the sample was assessed from the weight loss while holding the sample at 550 °C in air for 30 minutes. Both polymer in biomass and extracted PHA resins were quantitatively assessed by this standardized method. The weight loss and the rate change of weight loss as a function of temperature were considered. These trends informed on the thermal decomposition temperature ($T_d$) of the PHA-in-biomass, the PHA $T_d$ after extraction, the PHA content of the biomass, and the extracted PHA purity. The measurements were performed on a Q500 from TA Instruments. Criteria for thermal stability (decomposition temperatures) of recovered PHA and PHA-in-biomass are made with reference to this method of TGA.

### Biomass Characterization by FTIR Spectroscopy

Finely ground dried PHA-rich-biomass and PHA samples of between 2 and 10 mg were characterized by Fourier transform infrared spectroscopy (FTIR). FTIR spectra were collected and further analyzed using multivariate statistical methods (Partial Least Squares and Principal Component Analysis) of the spectral content for determination of the presence of PHA and discrimination of impurities influencing the chemical stability of PHA-in-biomass. The equipment used was a Bruker Alpha FTIR spectrometer equipped with an attenuated total reflection (ATR) diamond. The FTIR resolution used was 4 cm$^{-1}$, in the wave number range of 400-4000 cm$^{-1}$, and 24 scans per measurement were made with similarly acquired background scans for atmospheric compensation. The samples were loaded in the ATR, and each sample was then mixed and rescanned in triplicate. Data were collected using Bruker OPUS software and the data were then processed in OPUS or else with numerical or statistical analytical tools written for MATLAB.

### Size exclusion chromatography (SEC).

Molecular weight distributions (referenced to polystyrene standards) of the extracted polymer were determined by size exclusion chromatography (SEC). The SEC was performed with a pump (Viscotek VE 1122), a dual refractometer/viscometer-detector (Viscotek Model 250) and three linear columns coupled in series (Shodex KF-805, Shodex KF-804 and Shodex KF 802.5). The detector temperature was controlled to 37 °C, while the measurements were otherwise carried out at room temperature. The carrier solvent was chloroform (Merck pro analysis >99%) with a flow rate of 1 mL/min. The sample injection volume was 200 µl.

Molecular weight was calibrated with reference to four different polystyrene standards with known molecular weight averages of 650, 96, 30.3 and 3.18 kg/mol, respectively. Refractive index (RI) was used to for calibration of standards and assessment of samples.

Samples of PHA were dissolved in chloroform to a concentration of 5 mg/mL at 100 ºC for 10 minutes. Before sample injection, the polymer solutions were pre-filtered (PALL Life Sciences Acrodisc ® CR 25 mm Syringe Filter with 0.45 pm in pore size). From the logged RI as a function of elution volume, PHA molecular weights were characterised in terms of weight average molecular mass ($M_w$), number average molecular mass ($M_n$) and polydispersity index (PDI).

**Dynamic Viscosity Measurement by Rheology**

Polymer samples of 0.5-0.6 g were pressed at room temperature into 25 mm diameter discs. The polymer discs were mounted into a rheometer (TA Instrument AR 2000 or Discovery HR-2) and the dynamic viscosity was measured with a time sweep of 40 minutes at 180, 185 or 190ºC after an initial melt time of 8 minutes. During the time sweep, a constant 2% strain with a 10 Hz frequency was maintained. The temperature was maintained constant with nitrogen gas cooling.

The trend of the logarithm of polymer dynamic viscosity ($|\eta^*|$) in Pa s is used to estimate $|\eta^*|$ at a melt time of 4 minutes and this value is calibrated linearly to the weight average molecular mass ($M_w$). Such calibration was found to be valid so long as the melt could be considered to be non-Newtonian:

$$M_w = m \cdot |\eta^*|_{t=4} + b \qquad \text{EQ-E1.1}$$

The parameters m and b are constants that are dependent on the type of PHA and the conditions of the rheology measurement (temperature, frequency and strain). For the PHA produced in the examples presented m was 155.64 and b was 185729. These parameter values were calculated from regression analysis based on SEC data on representative samples. A decrease in $|\eta^*|$ is tantamount to a decrease in $M_w$. During rheology measurements decreases in $|\eta^*|$ of the polymer in the melt over time relate to $M_w$ decrease. For a given polymer where the constants m and b are anticipated to be the same, differences in $|\eta^*|$ indicate for proportional differences in the polymer $M_w$.

**One-Liter PHA-rich Biomass Extraction**

Dried (<5 % water w/w) and ground (nominal 0.71 - 2.00 mm, 0.71 - 3.15 mm, or 2.00 - 3.15 mm particle size distribution) PHA-rich biomass was loaded at room temperature and with stirring into a woven mesh basket (0.5 mm mesh) immersed in a pressure vessel (Büchiglas Uster Versoclave type 3E/1.0) filled to 1 liter with a PHA-poor solvent. Typically for the presented examples, 2-Butanol (Sigma-Aldrich ReagentPlus) was used as the reference extracting PHA-poor solvent. Loadings of PHA for extraction were made with respect to the measured biomass PHA contents. PHA-in-biomass content was evaluated by TGA. Typically, vessel loading of PHA-in-biomass corresponded to levels for reaching selected theoretical maximum concentrations of PHA-loading to the solvent of up to 100 g-PHA/L. The pressure vessel was sealed and the vessel temperature was set to reach selected targeted maximum temperatures in as short a time as possible. The solvent with immersed biomass was maintained with constant mixing at an average temperature, for pre-determined times in the order of minutes to hours, above a defined extraction temperature limit $T_{L15}$. Temperature, heating power, reactor pressure, mixing rate, and mixing torque were logged over the course of the extraction process.

Upon the pre-determined extraction time the PHA-rich solvent was expelled due to vapour pressure while the biomass was retained almost exclusively in the porous basket. In the exit line, an additional inline filter (0.1 mm mesh) removed smaller biomass particles that may have been present or else formed during the process of extraction. The hot PHA-rich solvent was disposed to a beaker and maintained at room temperature and the solvent was permitted to gel with or without mixing. After gelation the solvent was expressed from the polymer by physical pressing of the mass in a well-sealed cylinder maintained with an adjustable contact clearance to the cylinder base plate. This clearance at the base of the cylinder provided for a means to provide for a sufficient opening from which expressed solvent could drain. Pressing separated most of the solvent. Any additional clean-up of the polymer was achieved by homogenizing the pressed PHA cake in water or solvents in which case the cleaned filter cake was collected by filtration. The filter cake was dried at 70 °C (Binder FD Series 231).

**Ten-Liter PHA-rich Biomass Extraction**

Dried (<5 % water w/w) and ground (nominal 0.71 - 2.00 mm. 0.71 - 3.15 mm, or 2.00 - 3.15 mm particle size distribution) biomass containing PHA was loaded into a woven mesh basket (0.5 mm mesh) placed in a pressure vessel (BüchiGlas Uster kiloclave type 3E/12 lt.) containing 10 L of 2-Butanol (Sigma-Aldrich ReagentPlus), Biomass was typically loaded so as to achieve selected maximum theoretical PHA-rich solvent concentration ranging up to 100 g-PHA/L. The solvent was constantly passed through the biomass in the reactor by means of an external recirculation loop driven by a positive displacement pump (Wright pump TRA10 Series) operated at constant pumping rate. The resulting volumetric flow rate, which was dependent on pumping rate and liquid viscosity, was monitored (Endress+Hausser Proline Promass 80P) throughout the extraction. The liquid viscosity was dependent on solvent temperature, polymer concentration and polymer molecular mass.

The pressure vessel was closed during extraction in order to maintain the vapour pressure and prevent boiling. The temperature was ramped up to a selected maximum while the progress of the extraction was monitored and logged. With 2-butanol, extractions of PHB were achieved with maximum temperatures ranging between 120 to 160 °C. Vessel temperature, heating power, pressure and recirculation volumetric flow rate were logged. FT-NIR monitoring of PHA in the solvent (Bruker Optics Matrix-F FT-NIR with a IN1237P fiber optic probe) was made in the recirculation loop.

Upon the extraction, the PHA-rich solvent was released from the vessel, while withholding the biomass in the mesh basket. Discharged solvent was disposed through an inline trap (0.5 mm mesh) to a collection vessel. The PHA-rich solvent was cooled with or without mixing until the contents formed a physical gel.

After gelation, the solvent was expressed from the gel with mechanical force. Expressed solvent escaped by adjusting the clearance between the cylinder and the bottom base plate. Solvent exuded at the cylinder base with the polymer cake under pressure and the solvent was collected and recycled for reuse by evaporation. The pressed polymer cake could be homogenized and rinsed with solvent or water. Rinsed polymer was harvested by centrifugation or filtration. Ultimately, the polymer was brought to dryness by evaporation at 70 ºC.

**Example 2 - Kinetics of PHA Dissolution**

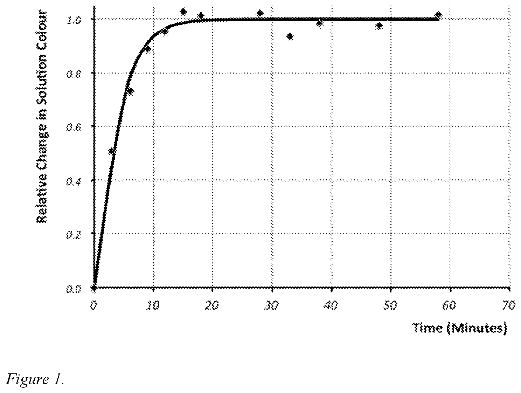

It was of practical interest to determine, as a benchmark, the native rate of dissolution of poly(3-hydroxybutyrate) (PHB) in a PHA-poor solvent. PHB was seen as a "worst case scenario." Due to the propensity for a higher crystallinity with PHB, it is understood to be harder to dissolve, especially in PHA-poor solvents. The dissolution trends of a fine pure PHB powder (> 98% purity) as a function of temperature were examined. PHB powder was weighed into 12 mL test tubes and combined with 5 mL aliquots of 2-butanol forming solutions of 50 g-PHB/L. The tubes were sealed with Teflon lined screw caps and were introduced into an isothermal heating block at selected temperatures. The tube contents were vortex mixed every three minutes and, directly after mixing, solution optical density and colour were measured. Relative changes in the solution colour were used to indicate for the progression of PHB dissolving in solution (Figure 1, Progress of PHB powder dissolving in Butanol at 132 °C based on measured changes of optical density and colour). Solutions were milky white powder solvent suspensions to begin with and translucent polymer rich solvent solutions to end with.

Over a relatively narrow temperature range (130 to 135 °C), came significant changes in the nature and kinetics of the dissolution process. In general, the dissolution of the PHA was observed to follow first order kinetics (Figure 1). However, a distinct lag in the dissolution process that appeared at 130 ºC was no longer evident by 132 ºC. Such a lag in dissolution may be interpreted to be related to a process of solvent infiltration that weakens the polymer crystallinity. The estimated first order dissolution kinetics increased by an order of magnitude from 0,3 to 3.7 $min^{-1}$ in moving from 132 to 135 °C. The trend of these data further suggested that temperatures in excess of 105 °C would be preferred in order to achieve any measure of the PHB dissolution for a PHA-poor solvent like 2-Butanol.

In this manner, the practical limits of extracting any particular PHA homo- or copolymer with any particular PHA-poor solvent may be assessed. Critical temperature levels that bring a shift in dissolution kinetics may be determined. Crystallinity of the PHA-in-biomass, the polymer crystallization potential, the PHA molecular weight, and the granule particle size distribution may all influence the extraction temperature-time-concentration conditions for a particular recovery process.

Ultimately, for too high PHA concentrations in the solvent, the associated increased solution viscosity may introduce practical process limitations. Since increasing extraction temperatures, times and PHA concentrations may increase the polymer degradation, it was anticipated that conditions would need to be selected in order to obtain a compromise between high extraction yield and criteria of acceptable recovered polymer quality.

**Example 3 - Kinetics of PHA Extraction**

Kinetics of PHA extraction from biomass may be limiting due to the polymer dissolution rate and/or due to the mass transport rates of solvent in and polymer out of the biomass. In order to better understand the kinetics of extraction, we examined idealized extraction conditions with finely ground biomass and over a range of temperatures. Samples (nominally 150 mg) of a finely ground PHB-rich biomass were weighed into 12 mL test tubes. The mass fraction of PHB in the biomass was estimated by thermogravimetric analysis (TGA). The nominal PHB content of the biomass was 50% w/w. The biomass was combined with 10 mL of 2-butanol and the tubes were sealed with Teflon lined screw caps, The theoretical maximum PHA loading to the solvent was approximately 7 g/L in these tests, A relatively low PHA loading was used for these trials for a reduced driving tendency for gel formation in order to facilitate easy biomass separation from the PHA-rich solvent in this model test-tube-scale benchmarking experimental system.

Isothermal extraction for a fixed time was performed at selected temperatures from 125 to 140 °C with the sealed tubes in a thermostatic heating block. The tube contents were vortex mixed every 5 minutes during fixed extraction times of either 15, 30, or 45 minutes. At the end of the respective extraction times, the tubes were permitted to cool for 3 minutes allowing for the residual biomass to settle and for the overpressure to become sufficiently decreased without the onset of gel formation prior to removing the screw cap. Most (about 95 %) of the hot solvent was carefully poured out into a clean petri dish while retaining the residual biomass in the test tube. By evaporating the residual solvent in the tube and assessing the weight loss due to this solvent evaporation, the volume of residual solvent remaining with dissolved PHA in the tube was accounted for. The mass of the dried extracted material captured in a petri dish was weighed and the fraction of PHA in the extracted material was determined by TGA. From mass balance principles, the extracted fraction of the PHA from the biomass could be reproducibly estimated.

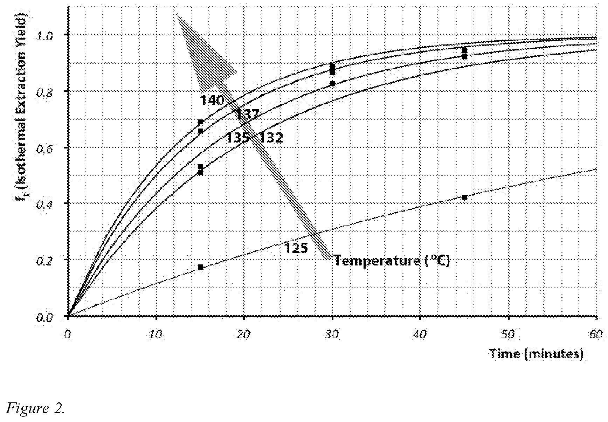

In replicate experiments, the PHA extraction trend was observed to follow the model of first order kinetics (Figure 2 - Modeled first order isothermal extraction kinetics as a function of temperature for a PHB-rich-biomass). PHB-rich biomass derived from distinctly different pilot plants operating in Belgium and Sweden were tested. In one such pilot plant, biomass was produced due to treatment of organic matter in a municipal wastewater (Belgium). In the other pilot plant, the biomass was produced due to treatment of organic matter discharged from a food processing industry (Sweden). The biomass from these sources was made to be PHB-rich by controlled feeding of acetic acid in an accumulation process applying the principles described in WO2011/070544A2. PHB was selected as a worst-case scenario as PHB is generally more resistant to dissolve in PHA-poor solvents. This particular challenge with PHB may be due to a propensity for higher crystallinity of the PHA-in-biomass. A superior PHA-in-biomass thermal stability ($T_d \approx 285°C$) was achieved by applying methods described in WO2012/022998A1 prior to these solvent extraction experiments.

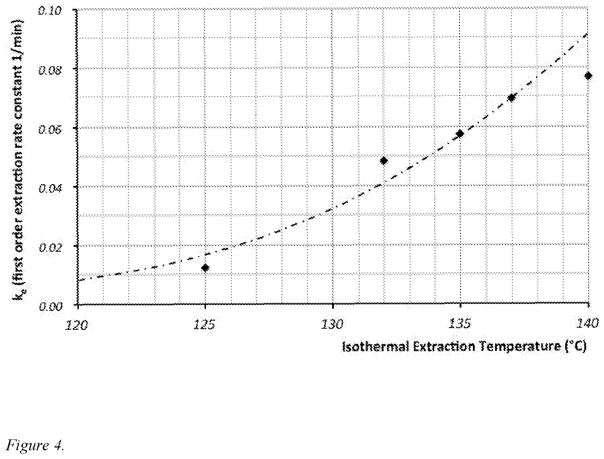

In contrast to the PHB dissolution kinetics of Example 1, the extraction rate from a finely ground PHB-rich biomass was almost two orders of magnitude slower. At 135 $\underline{o}$C, the first order extraction rate constant was approximately 0.05 min$^{-1}$ (Figure 4 - Empirically estimated first order isothermal extraction rate coefficient as a function of temperature and the modeled values (line) based on data of $f_{15}$ as a function of temperature). Therefore, mass transport of the polymer out of the biomass and into solution was rate limiting to the extraction process relative to dissolving just PHB alone (Example 2). Furthermore, the extraction kinetics were observed to be temperature dependent. One may consider that temperature dependent viscosity of a polymer-rich solvent in the biomass will impede the transport of polymer out of the porous biomass structure especially at lower temperatures and higher concentrations. Notwithstanding the possibility for a number of physical interpretations of these data, increasing temperature generally improved the extraction rates.

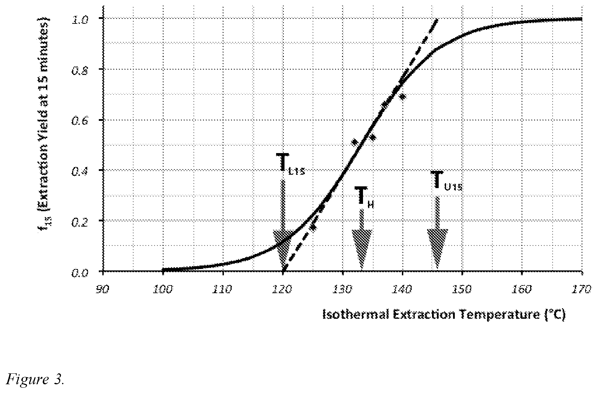

For a given PHB-rich biomass, the fraction of extracted PHB for a fixed extraction time of 15 minutes followed a trend as a function of temperature in the experiment range from 125 to 140 °C. Fifteen minutes was found to be a suitable time length for the purpose of this particular assessment because by 15 minutes some but not all polymer was generally extracted in the temperature ranges tested. For other biomass, particle size distributions, and/or PHA-type in the biomass, other assessment times may be more suitable as described in more detail below. The observed trend was modelled empirically as a sigmoid function (Figure 3 - Experimental results for 15-minute isothermal extraction from a PHB-rich-biomass with model sigmoid fit with extrapolated line from $T_H$ to operational limiting temperatures of $T_{L15}$ and $T_{U15}$) that described the trend of increased extent of extracted polymer with temperature after 15 minutes:

$$f_{15} = \frac{1}{1 + e^{-k_{15}(T - T_H)}}$$

EQ-E3.1

where

f $_{15}$ = fraction of extractable polymer after 15 minutes as a function of Temperature (T)
$k_{15}$ = 15 minute extraction rate temperature coefficient
$T_H$ = the temperature tor $f_{15}$ equal to 0.50.

We used the slope of the modelled $f_{15}$ sigmoid at $T_H$, and the line extrapolated therefrom, in order to define two practical operational temperatures for the extraction process. An extraction temperature limit $T_{L15}$ was defined as the temperature below which negligible polymer would be expected to be extracted from the biomass after 15 minutes ($f_{15}(T_L) \approx 0$). Further we defined the upper limit temperature $T_{U15}$, where after 15 minutes, most of the polymer should be extracted ($f_{15}(T_U) \approx 1$).

By way of illustration of batch-to-batch variability for different PHA-rich biomass batches, all with the same type of PHA (PHB), from 13 different experiments with different biomass samples, the 15 minute PHB extraction trials suggested an average $T_{L15}$ of 119 ± 6 °C. A component of the experimental variation was believed to have been due to differences in the condition of the PHA in the biomass. For example, it is known that the polymer degree of crystallinity will influence the extraction process with PHA-poor solvents. In general, the time taken for the solvent to infiltrate the polymer-in-biomass may be expected to be influenced further by factors such as the copolymer blend composition, average molecular weight, intracellular granule density and size distribution, and biomass particle size distribution.

Notwithstanding, the procedure described herein provides for a means to pragmatically characterize and adapt, where appropriate, to the extraction kinetics for a biomass with industrial scale PHA extraction implementations. In the above example of 13 different experiments with PHB-rich-biomass, a $T_{U15}$ of 150 ± 7 °C was estimated.

Given the observation of first order rate of extraction under isothermal conditions we estimated, by example, the polymer first order extraction rate coefficient from the fixed time isothermal extraction data (Figure 4);

$$k_e = \frac{-1}{t} ln(1 - f_t)$$

EQ-E3.2

where

    $k_e$ = the estimated first order temperature dependent extraction rate constant (1/min).
    $t$ = time in minutes used for the isothermal extraction characteristics,
    $f_t$ = the extraction yield at time t.

Notwithstanding, other analogous approaches or other time frames may be used to estimate the influence of temperature on the kinetics of PHA extraction from biomass.

In a larger scale extraction process the biomass may be exposed to a range of temperatures as a result of the length of time that may be utilized to heat the solvent. Given knowledge of the extraction temperature history the extraction progress may be estimated as follows:

$$\frac{df}{dt} = k_e(1 - f)$$

EQ-E3.3

where

    $f$ = the fraction of extracted PHA (0 < f < 1 ).
    $k_e$ = the temperature dependent first order extraction rate constant.

Such a predictive model can be further adapted to compensate for differences between estimated and industrial scale extraction kinetics. Differences in predicted and actual kinetics may be due, for example, to less than ideal mixing and mass transfer for an industrial scale process. The methods described herein provide for a means to estimate and adopt extraction time limits for the process control that are compatible with the temperature conditions of extraction, the solvent, the type of PHA, the biomass granule particle size distribution, and the granule properties (density).

It was recognized that the process parameters for extraction may vary significantly with the type of PHA in the biomass. It is also detrimental to expose the PHA to unnecessarily higher temperatures or longer extraction times if the polymer may be recovered at lower average extraction temperature and/or for shorter times. The PHA-rich biomass may contain homogeneous or heterogeneous blends of PHB, and/or PHBV. To this end we examined the 15 minute extraction behaviour of distinctly differently produced PHBV-rich biomass over selected temperatures in the range from 70 to 140 °C. The extraction kinetics followed the sigmoidal model but with at least two distinct regions of extractable PHA fractions. In one case, approximately 76 percent of the PHA was characterized by a $T_{L15}$ of 53 °C and a $T_{U15}$ of 86 °C. The remaining 24 percent of the PHA was found to exhibit a $T_{L15}$ of 98 °C and $T_{U15}$ of 135 °C. A second PHBV-rich biomass also exhibited at least two distinct fractions with both in keeping with the sigmoidal model. In this second case, approximately 80 percent of the PHA was represented by a $T_{L15}$ of 58 °C and a $T_{U15}$ of 86 °C. The remaining 20 percent of the PHA was with a $T_{L15}$ of 91 °C and $T_{U15}$ of 135 °C. Thus, a particular biomass may contain distinct fractions of PHA, which follow independent temperature dependent extraction kinetics. It follows that the above predictive model can be generalized to PHA-rich biomass with distinctly different regions of homogeneously extractable fractions:

$$\frac{df}{dt} = \sum_{i=1}^{n} \frac{df_i}{dt} = \sum_{i=1}^{n} k_{ei}(a_i - f_i), \ \sum_{i=1}^{n} a_i = 1 \quad , f_i \le a_i \qquad \text{EQ-E3.4}$$

where

f$_i$ = the i$^{th}$ fraction of extracted PHA ($0 <= f_i < a_i$),
$k_{ei}$ = the i$^{th}$ fraction's 1$^{st}$ order extraction rate constant.

Where the objective is to extract all of the polymer from the biomass, then the conditions and times of the PHA recovery would need to be tuned to the hardest to extract fraction of the PHA blend in the biomass. In other words, the highest $T_{L15}$ and $T_{U15}$ pair would be applied.

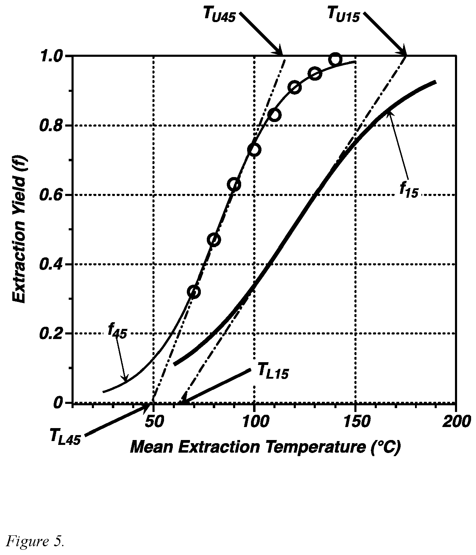

As an illustrative example, a PHA-rich biomass (identified herein as CFS16) was produced containing a copolymer, Poly(3-hydroxybutyrate-co-3-hydroxyvalerate) or PHSV with average 43 wt.-% of 3-hydroxyvalerate (3HV) (measured with GC). The biomass was granulated with a particle size distribution between 0.71 and 2.00 mm. Based on a simple test tube laboratory scale experimental series, the isothermal extraction yields for this granulate were determined as a function of temperature based on a fixed extraction time of 45 minutes ($f_{45}$). The yield data (Figure 5 - Experimental results for 45-minute isothermal extraction ($f_{45}$) from a PHBV-rich- biomass with model sigmoid fit and data translated to operationally defined 15-minute extraction ($f_{15}$) with limiting temperatures of $T_{L15}$ and $T_{U15}$) were fit according to equation E3.1 and the 45-minute lower and upper temperature thresholds ($T_{L45}$ and $T_{H45}$) could be determined. Based on the assumption of first order extraction kinetics (Equations E3.2 and E3.3), the fit curve for $f_{45}$ could be translated to predict extraction yields as a function of temperature based on a fixed extraction time of 15 minutes ($f_{15}$). From the model curve of $f_{15}$, the reference temperature thresholds ($T_{L15}$ and $T_{H15}$) could be now estimated (Figure 5).

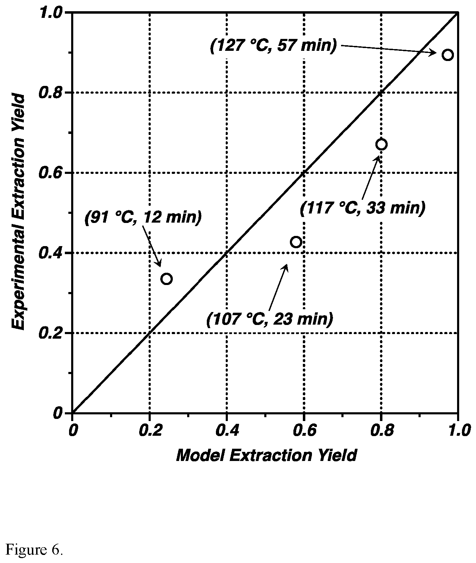

In practical trials using a 1 L extraction process (Example 1), the PHBV was extracted in a series of batches varying the extraction times and average extraction temperatures above $T_{L15}$ of 62,7 ºC. The experimental extractions were compared to the expected values (Figure 6 - With reference to Figure 5, model versus experimental extraction yields from a PHBV-rich-biomass with average temperature and extraction times (T > $T_{L15}$) as indicated) based on the estimated first order extraction kinetic constants (Figure 5). The experimental data follow the expected values as a whole but there were also differences. The model expectations under estimated the yields at lower temperatures and overestimated the yields at higher temperatures. The assessment (Figure 5) provided values for the PHA in the biomass as a whole. However, thermal characterization of the PHBV in CFS16 suggested that this co-polymer blend melts over a temperature range suggesting a blend of PHBV co-polymers. Most of the polymer melts below 120 °C but a fraction of the polymer melts between 160°C and 170°C. The low melting co-polymer fractions in the blend are more readily extractable than the higher melting fractions.

**Example 4** - **The influence of biomass particle size on extraction performance.**

When biomass after an accumulation process is dewatered to a dry solids content in excess of approximately 15% and preferably in excess of 20% it may take on a consistency of a formable solid like dough. In our experience, semi-dry biomass can be formed, machined, or extruded and dried into particles of different shapes and sizes. The dried particles can also be broken down mechanically into defined particle distribution windows. These dried biomass particles are generally brittle but they may retain their basic form during the extraction process. The basic particle form is retained so long as the mechanical forces experienced by the particles during the extraction process are not too aggressive.

It is preferred that fine biomass particles do not end up in the recovered polymer after extraction. Therefore, the more effective the biomass separation from PHA-rich solvent is, the better the as-extracted product quality will be. In order to simplify physical separation of residual biomass from the PHA-rich solvent, we found that larger particle size is preferable, and this generally meant particle sizes greater than 0.1 mm, but preferably greater than 0.6 mm. However, based on the results presented in Example 2, we understood that mass transport of the polymer out of the biomass was rate limiting and influenced by temperature. Therefore, unnecessarily too large particles were also seen to be undesirable for the extraction process. Thus, in preferred embodiments, as described below, mean particle sizes may be less than 4 mm but preferably smaller than 2 mm.

We found that biomass particle sizes greater than 0.5 mm were easy to separate from the PHA-rich solvent using simple methods of filter screen separation during the PHA-rich solvent discharge. When a solvent boiling point is less than the extraction temperature, then the vapour pressure formed in the closed extraction system was found to be effective to drive the PHA-rich solvent through a filter screen, leaving the solid residue behind. While particle dimensions larger than 0.5 mm would simplify process operations of separating the PHA-rich solvent from the spent biomass, the question was how large could the particle size be. A balance aids in facilitating mass transport (smaller biomass particles) and ease of PHA-rich solvent separation (large biomass particles). To this end extractions were performed at conservatively low temperature (125 °C for 1 hour) in 2-butanol with exaggerated large biomass particles (nominally 5 mm). Based on the kinetic rate constants for a fine PHB-rich biomass powder (Example 3), we expected to achieve approximately 50 % extraction after 1 hour at 125 °C if polymer transport out of the particle was not impeded.

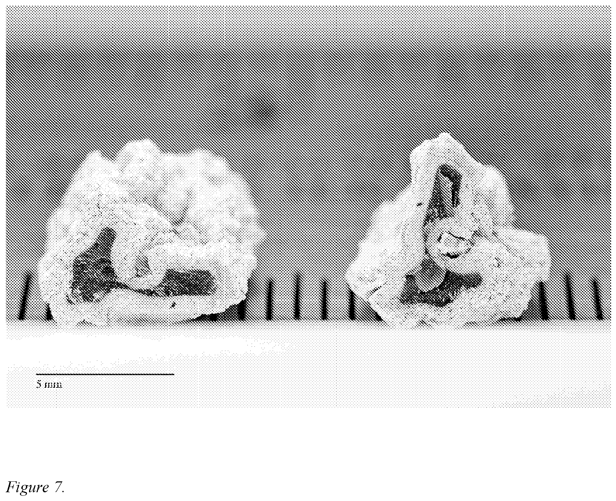

When these larger particles were subsequently dissected (Figure 7 - Influence of particle size on extraction of PHA-rich-biomass), the solvent penetration depth was visible due to discoloration through the cross section. The penetration depth varied and such variation may have been due in part to localized differences in the material porosity. However, the penetration depth was generally equal to or larger than 1 mm, typically less than 1.5 mm, and never larger than 2 mm (Figure 7).

Samples of the discoloured surface and core were analysed separately by TGA in order to confirm the spatial differences in the extent of extraction. The degree of extraction was estimated with respect to the original content of PHA in the biomass. In agreement with expectations, we found that the degree of extraction for the outer particle surface was 49% with respect to the organic weight of the particle. There was no measureable PHA extraction for the darker particle core. However, a slight but significant decrease of the inorganic content of the core suggested some very minor degree of dissolution of matter from the core of these larger particles during the extraction process.

The influence of particle size distribution was further investigated by direct measurement on a PHA rich biomass given distinct particle distributions of the granulated biomass. Large dried biomass pieces of varying sizes were fed through a 3-roll mill crusher. The roll mill cylinders (150 mm in length and 38 mm in diameter) in the feeding section were placed 3.0 mm apart and the third crushing cylinder was adjusted to a position giving a nominal clearance of 1.5 mm. After the biomass was crushed through such a device, the biomass particle size distribution was evaluated by means of a Retsch AS 200 basic sieve shaker having six sieve trays with decreasing mesh sizes of 3.15, 2.00, 1.60, 1.00, 0.85. and 0.71 mm.

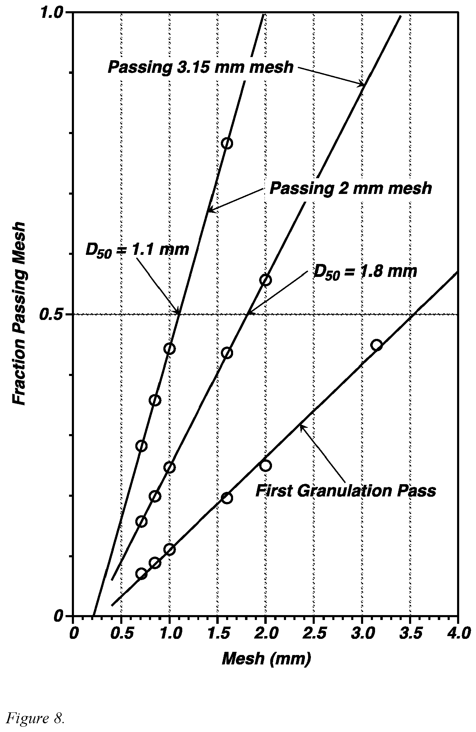

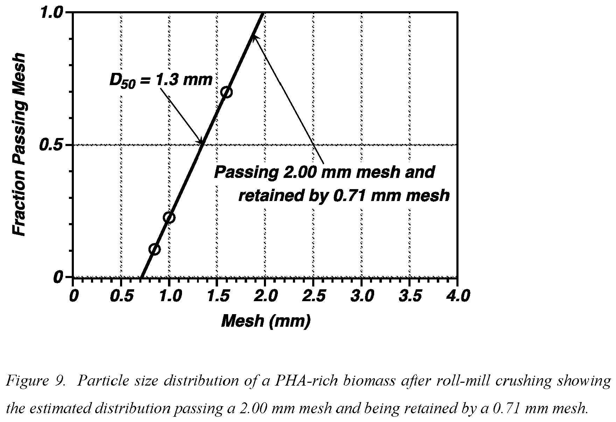

From mass balance considerations, the particle size distribution could be approximated, for which granulated biomass passing through such a mill, and then screened by the 3.15 mm mesh exhibited a $D_{50}$ of approximately 1.8 mm and a particle size distribution from 0.2 to 3.4 mm (Figure 8 - Particle size distribution of a PHA-rich biomass after roll-mill crushing showing the results for the first milling pass of the biomass and the distribution of selected sub-fractions). Extractions were made on such crushed biomass passing the 2.00 mm sieve and retained by the 0.71 sieve. Here, the nominal $D_{50}$ was about 1.3 mm and the estimated particle size ranged from 0.7 to 2.0 mm (Figure 9 - Particle size distribution of a PHA-rich biomass after roll-mill crushing showing the estimated distribution passing a 2.00 mm mesh and being retained by a 0.71 mm mesh). For comparison, identical extractions were made with crushed biomass passing the 3.15 mm sieve and retained by the 2.00 mm sieve. Here, the nominal $D_{50}$ was approximately 2.7 mm with an estimated particle size distribution between 2.0 mm and 3.4 mm. In one such example, a PHA-rich biomass containing 39% PHA with a $T_{L15}$ of 78 °C was extracted for 41 minutes above $T_{L15}$ at an average temperature of 129 °C. The PHA recovery yields with the estimated $D_{50}$ of 1.3 and 2.7 mm particle size distributions for the same biomass were approximately 87 and 67 percent. In a second example, a PHA-rich biomass containing 43% PHA with a $T_{L15}$ of 63 °C was extracted for about 43 minutes above $T_{L15}$ at an average temperature of 125°C. The yields with an estimated $D_{50}$ of 1.3 and 2.7 mm particle size distributions for the same biomass were approximately 81 and 68 percent. Thus, particle size distribution is important to the process performance, and these results suggest significant benefits of preparing the granulated biomass with a $D_{50}$ that is below 2 mm.

From these results, we have found practical limits in biomass particle size for balancing considerations of extraction yield (mass transport) and product (PHA-rich solvent) separation. The dominant particle size distribution should be between 0.1 and 4 mm, but more preferably between 0.5 and 2 mm.

**Example 5 - PHA-In-biomass Molecular Weight Determination**

This preferred embodiment addresses a need to be able to extract a PHA from a biomass in a process with tuned batch-to-batch conditions of solvent-time-temperature-loading so as to consistently obtain a predetermined recovered product quality. It was found that the chemical stability of the polymer is sensitive to the total biomass loading to the solvent. The biomass loading to the solvent can be divided up in terms of PHA-in-biomass loading and non- PHA-biomass loading. Both the concentration of PHA and the concentration of non-PHA biomass constituents may influence the polymer scission rate during extraction. The conditions of solvent-time-temperature-loading for a particular total biomass loading will result in a specific scission rate for the polymer during extraction. The number of scissions by the end of the extraction process determines how much the polymer chains will become cut on average. The average number of polymer chain scissions translates to a decrease in polymer molecular mass from an initial value. Thus, the initial molecular weight distribution of the PHA-in-biomass determines just how many scissions may take place if the recovered polymer is to consistently meet defined product quality standards of molecular mass.

For example, consider the case for a particular polymer application where it is specified by the customer that the polymer to be processed must exceed a number average molecular mass of 200 kDa. If the PHA-in-biomass is with a number average molecular mass of 300 kDa, then by equation EQ-1, the extraction process must not result in a scission number of more than 0.5. If the PHA-in-biomass number average molecular mass were 400 kDa, then the maximum tolerated scissions for extraction would be 1.0. With all other factors being equal, the latter case permits for a greater biomass loading to the extraction process while achieving the same end product quality. Selected extraction conditions of time-temperature-solvent matched to suitably selected biomass loadings regulate the product quality.

It follows that a conservative estimate of the PHA-in-biomass molecular weight is useful information towards selection of the appropriate biomass loading conditions. Without limitation to the possibility of a multitude of possible strategies for such an assessment, representative approaches for conservative reference methods of PHA-in-biomass molecular weight evaluation are presented herein. These methods are protocols intended to either extract PHA under conditions known to impose reduced loss of molecular mass or where the loss of molecular mass can be accounted for so as to estimate the PHA-in-biomass molecular mass average. For example, PHA is extracted from powdered biomass at the test tube scale using acetone and a nominal PHA loading of 10 g-PHA/L or less. Extractions are for 1 hour at 125 °C in a heating block with vortex mixing of the test tubes at least once every 15 minutes. After reaching the set extraction time the test tubes (typically 6 tubes with 10 mL solution each) are allowed to cool for 3 minutes at room temperature before pouring PHA-rich solutions into a 50 mL beaker.

Deionised water is added drop wise until the polymer precipitates and the polymer is then harvested from solution by vacuum filtration and dried at 70 °C. Sample quality is confirmed by TGA and rheology, Molecular weight is quantified by standard methods of rheology and/or size exclusion chromatography.

As an alternate, butanol is used with an extraction temperature of 140 °C for fifteen minutes and a nominal PHA loading of 10 g-PHA/L or less. Polymer-rich solution is decanted following the methods of Example 3. Product quality and molecular weight are assessed as above.

The combination of either maintaining lower temperature or lower time, together with a lower biomass loading, results in a reference material to provide for an estimate of the polymer molecular weight in the biomass. It is recognized that some molecular weight loss of the polymer may ensue even in such a standard extraction method. The estimate is therefore conservative because molecular weight loss as part of this assessment will tend to lead to an underestimation of the allowable maximum biomass loading for the extraction process.

A more rigorous method is to perform a series of extractions at the test tube scale varying either extraction time or loading. In each case, the molecular weight is assessed, and based on the trend as a function of time (or loading) the PHA-in-biomass average molecular mass can be estimated from the trend by extrapolation the point of zero time (or negligible loading).

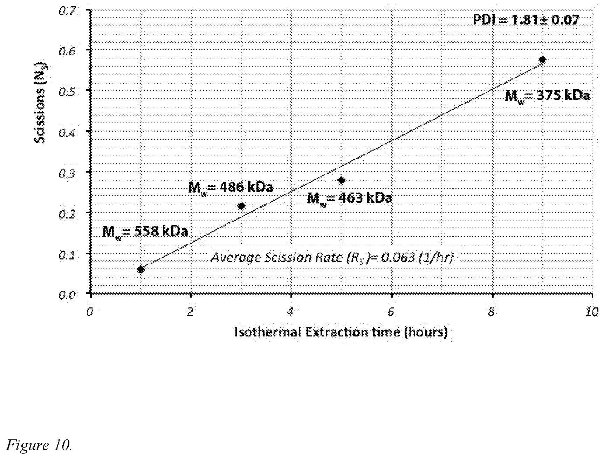

In one example (Figure 10 - Average scission rate for isothermal extractions (125 ºC) of a PHB-rich-biomass in 2-butanol at constant biomass loading), where a standard extraction method predicted a PHA-in-biomass weight average molecular mass to be 561 kDa, the trend of a control series with constant loading and varying extraction time from 1 to 9 hours suggested a PHA-in-biomass $M_w$ of 591 kDa. These results also served to illustrate a repeatedly observed constant scission rate under isothermal conditions for a given biomass loading.

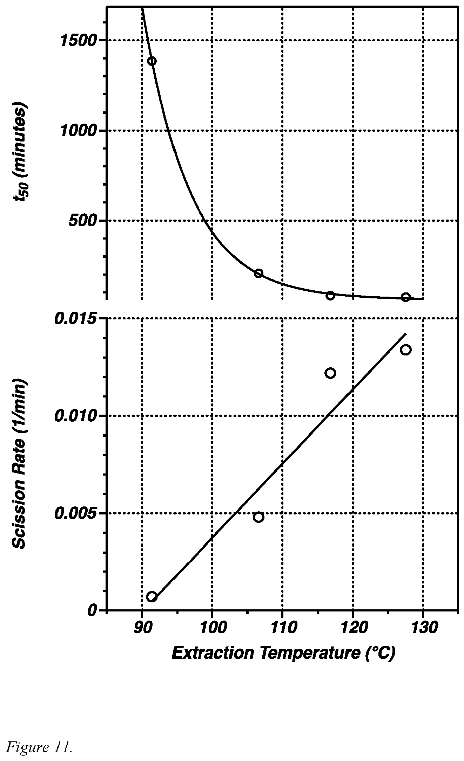

With reference to CFS16 presented in Example 3 (Figure 5), the PHA-in-biomass $M_w$ was estimated to be 611 kDa. Thus from the practical extraction trials that were performed (Figure 6), the product molecular weight was determined and the scission rate as a function of temperature was estimated for the case of an extraction PHA-loading of 50 g/L (Figure 11 - Average scission rate as function of average extraction temperature for a PHBV-rich-biomass in 2-butanol at constant PHA-loading of 50 g/L. The time $t_{50}$ is the estimated extraction time resulting in a 50% loss of average molecular mass). Due to the scission rate as a function of temperature, one may expect that available extraction time will decrease as a function of temperature given a specific target product molecular weight. Figure 11 illustrates the exponential decrease in available extraction time with constant resultant scissions for a 50 percent loss in average molecular mass ($t_{50}$ in minutes).

Consider for example that the product molecular weight is to be 400 kDa or greater.

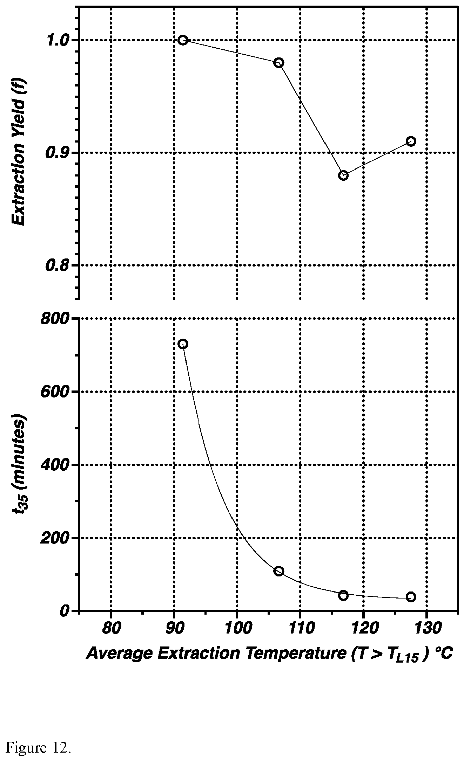

Based on the scission rate as a function of temperature for a 50 g/L PHA-loading, the time available for 35 percent loss in average molecular mass, and the associated predicted extraction yield can be estimated (Figure 12 - Allowable extraction time based on a PHA-loading of 50 g/L for a fixed 35 % loss of molecular mass ($t_{35}$) influences the expected yield for the extraction (f)). Predictions can be confirmed and the model improved from practical trials. The shorter allowable extraction time for the higher average temperatures may not provide enough time for the highest possible extraction yield, However, lowering the PHA-loading generally deceases the scission rate, and this allows for a longer allowable extraction time for the same average extraction temperature.

**Example 6** - **Introducing Additives to The Extraction Process**

Chemical additives may be introduced to the process towards improving productivity and/or manipulating the final product quality. Additives may be brought into the extraction process when the hot solvent is in contact with the biomass. Alternatively, additives may be introduced to the PHA-rich solvent after biomass separation but before gelation. Blending of additives into the mixture during or after gelation is a third opportunity for chemical addition, The underlying motivations for chemical additions may be varied. Without limitation, a number of examples are provided herein for illustration.

The loading of biomass into the extraction process may be constrained by the allowable number of scissions. Chemical additives that act to hinder the reaction of random chain scission are of potential value to enable a higher biomass loading. For example, the scission rate of the polymer is influenced by the presence of cations. Additives to the biomass that sequester cations therefore may help to hinder chain scission reactions. Similarly, moisture increases the scission rate. Biomass is hygroscopic and we have typically observed that biomass after drying equilibrates upon storage to moisture contents in the order of 2 to 5 percent, We found that pre-drying of the biomass directly before extraction effectively reduced scission rate by as much as 25%. Therefore, one strategy to limit the influence due to the presence or generation of water during the extraction process would be to include a water scavenger with the biomass during extraction.

One advantage of bringing polymer additives to the PHA-rich solvent after biomass separation relates to the ease in which such additives can be rapidly and homogenously dispersed in the matrix. An alternative would be to combine such additives during melt processing but melt processing involves high temperatures and risks an undesirable degree of material degradation in the blending. Blends of PHA can be combined as PHA-rich solutions or gels of different co-polymers from different extraction batches, The polymer can also be modified after extraction but before gelation by introducing reactive compounds and, therein, beneficial chemical reactions. For example, we have demonstrated this principle with a polycarbodiimide. The polycarbodiimide was provided by Rhein Chemie in Germany under the trade name Stabaxol® P. Stabaxol® P was in powder form and had a weight average molecular mass of approximately 3000g/mol with N=C=N content of 13%. Polycarbodiimide contains the highly unsaturated group -N=C=N-, which reacts with the carboxyl groups. The use of this additive as a chain extender in PLA systems has been well demonstrated.

Extraction of PHB from a biomass was performed with and without the presence of Stabaxol® P. The biomass containing 44 % PHB by weight was combined with 2-butanol towards generating a theoretical extracted PHA-rich solvent solution of 30 g-PHA/L. In cases with the polycarbodiimide, a 2% weight of Stabaxol® P was used where the weight percent was with respect to the extraction PHA loading. PHB was extracted from the biomass for selected times from 1 to 9 hours under isothermal conditions at 125 °C. The extraction efficiency was nominally 83 percent and independent of time, indicating that observed effective limit of extent in polymer extraction from this particular biomass was already achieved within the first hour for this extraction temperature.

Further it was observed that the presence of Stabaxol® P increased the scission rate by 32 percent from 0.06 to 0.08 $h^{-1}$. Notwithstanding the induced increased scission rate, the extracted polymer in the presence of the polycarbodiimide was predicted to promote chain extension. The extracted PHB was estimated to be with a significantly higher initial weight average molecular weight of 657 kDa compared to the predicted molecular weight of the control (591 kDa). Thus, a polycarbodiimide based additive will rapidly react in a beneficial way with the PHA in the PHA-poor extraction solvent at elevated temperatures. However, these benefits may not be prolonged if the reactive mixture is maintained at elevated temperatures for an extended period of time. Therefore, most appropriate time point to combine the polycarbodiimide with the polymer rich solvent solution would be after separating the biomass from the solvent and just prior to solvent cooling for gelation.

**Example 7** - **Co-extraction of non-PHA biomass and A Solvent Prewash**

PHA-poor solvents will extract PHA above a limiting extraction temperature for which we have defined without limitation a $T_{L15}$ temperature. In addition, non-PHA biomass will become extracted. We have assessed non-PHA biomass extraction in replicate test tube extraction experiments that were performed on batches of PHA-rich biomass. These PHA-rich biomass batches were derived from treating either municipal or industrial wastewaters.

Samples (nominally 150 mg) of a finely ground PHB-rich biomass were weighed into 12 mL test tubes. The mass fraction of PHA in the biomass was estimated by thermogravimetric analysis (TGA). The nominal PHA content of the biomass was 50% w/w. The biomass was combined with 10 mL of 2-butanol and the tubes were sealed with Teflon lined screw caps. The theoretical maximum PHA loading to the solvent was approximately 7 g/L. A very low PHA loading was used for these extraction trials for a reduced driving tendency for gel formation in order to facilitate easy biomass separation from the PHA-rich solvent in this model small-scale test tube experimental system. The PHA-rich biomass was extracted at selected temperatures (125, 132, 135, 137, and 140 °C) and selected times (15, 30, and 45 minutes), The purity of the extracted polymer was assessed by TGA and from mass balance considerations the fraction of the extracted non-PHA biomass was also estimated.

In addition, extraction trials at bench and pilot scale were conducted at 1 and 10 L volumes, respectively, with more significant PHA loadings ranging from 30 to 70 g/L. For these experiments, a known weight of biomass was introduced into the extraction reactor and polymer was extracted from the biomass for 29 (1 L) and 33 (10 L) minutes over an estimated nominal PHA-in-biomass $T_{L15}$ of 119 °C and with a peak temperature below 145 °C. The extraction-time-averaged temperature was 138 °C (1 L) and 135 °C (10 L). PHA-rich solvent was discharged from the reactor and cooled to gelation. Representative samples of dried PHA-rich biomass, residual biomass and solvent gel were assessed by TGA. From mass balance considerations PHA and non-PHA fractions were estimated as follows:

$$X = G + R \qquad\qquad \text{EQE7.1}$$

$$1 = x_p + x_n = \frac{X_p}{X} + \frac{X_n}{X} \qquad\qquad \text{EQE7.2}$$

$$1 = r_p + r_n = \frac{R_p}{R} + \frac{R_n}{R} \qquad\qquad \text{EQE7.3}$$

$$1 = g_p + g_n = \frac{G_p}{G} + \frac{G_n}{G} \qquad\qquad \text{EQE7.4}$$

$$Y_p = \frac{G_p}{X_p} = (1 - \alpha)\frac{g_p}{x_p} \qquad\qquad \text{EQE7.5}$$

$$Y_n = \frac{G_n}{X_n} = (1 - \alpha)\frac{1 - g_p}{1 - x_p} \qquad\qquad \text{EQE7.6}$$

$$\alpha = \frac{x_p - g_p}{r_p - g_p} = \frac{x_n - g_n}{r_n - g_n} \qquad\qquad \text{EQE7.7}$$

where,

X, G, R = mass of PHA-rich biomass, dried gel, and residual biomass
$X_p$, $G_p$, $R_p$ = mass of PHA in PHA-rich biomass, dried gel, and residual biomass
$X_n$, $G_n$, $R_n$ = mass of non-PHA in PHA-rich biomass, dried gel, and residual biomass.
$Y_p$, $Y_n$ = extraction yield of PHA and non-PHA biomass

Molecular weight of the extracted PHA was assessed by rheology and the average scission rates estimated with respect to reference PHA-in-biomass polymer molecular weight determinations (Example 5).

In general we found that the extracted amount of non-PHA biomass varied significantly in a range from 5 to 50 percent of the loaded non-PHA biomass. We observed that the amount of extracted non-PHA biomass correlated with the fraction of the non-PHA biomass volatilized during TGA at temperatures below 200 °C. The kinetics of non-PHA biomass extraction were influenced by temperature as on average the non-PHA content of the extracted material increased with temperature for a given extraction time. Notwithstanding the variability of non-PHA biomass extraction, the total amount of non-PHA extracted biomass did not, to our surprise, correlate with scission rate during extraction. However, aqueous rinsing a particular biomass batch reduced the non-PHA biomass fraction volatilized during TGA at temperatures below 200 °C and results suggested that this fraction of the biomass does contain components that do influence the extraction scission rate.

In order to better appreciate the potential for influence of non-PHA extractable material on the chemical stability of the PHA during extraction, we examined the effect of pre-treatment of the biomass with the extraction solvent. A series of test tube extractions were performed on a biomass containing 44 percent PHB. In this series, samples were first extracted for selected durations in butanol at 90 ºC (below the case specific estimated $T_{L15}$ of 119 °C) for up to 45 minutes. After this sub-$T_{L15}$ extraction, the butanol was decanted and the remaining biomass was extracted with a fresh aliquot of butanol using a PHB loading of 15 g/L for 5 hours at 125 °C. The PHA-rich solvent was decanted and the molecular weights were determined using SEC.

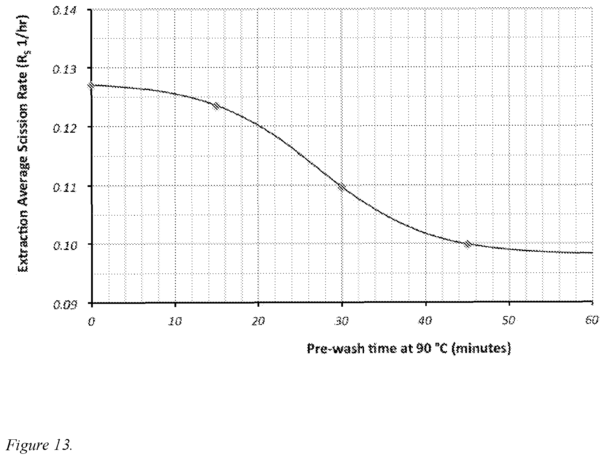

The polydispersity index of samples with varying degrees of average molecular mass loss was approximately constant ($1.76 \pm 0.06$). The scission rate was determined with respect to a referenced PHA-in-biomass polymer molecular weight determination (Example 5). Pre-extraction of non-PHA biomass resulted in a significant reduction in the observed scission rate. Thus, significant improvement of the polymer chemical stability was achieved with increased pre-extraction time. Up to an estimated maximum reduction by 23 percent of the average scission rate without pre-extraction was achieved (Figure 13 - Influence on extraction scission rate of a 90 °C pre-wash with 2-butanol for constant loading of a PHB-rich-biomass).

The efficacy of the solvent pre-treatment to reduce scission rate was further evaluated in replicate experiments at larger scale. In one example, 1 L butanol extractions were performed with a biomass containing 62 % PHB. The extraction was undertaken with a PHB loading of 50 g/L. When the biomass was processed with pre-extraction at 90 °C for 45 minutes before PHB extraction for 29 minutes at 138 °C, the scission rate during extraction was reduced by 47 percent.

Thus, we found that, although the total extractable non-PHA biomass, as a whole, does not influence the polymer stability, the biomass does nevertheless contain non-PHA extractable compounds which do influence scission rate. The influence of these compounds on PHA recovery can be mitigated by their separate selective extraction, at temperatures less than the lowest $T_{L15}$ for the PHA-rich biomass co-polymer blend, in advance of PHA extraction.

Further benefit of the pre-treatment is to remove non-PHA material that would otherwise become associated with PHA in the gel after extraction. It was evident from these investigations that varying degrees of processing after gelation may be appropriate, as a result of different biomass batches, in order to arrive at similar levels of sample purity. Furthermore, the polymer chemical stability is due, at least in part, to extractable compounds that may be present to varying degrees in different batches of PHA-rich biomass.

**Example 8** - **Assessment of PHA-rich Biomass Loading**

Example 5 describes a methodology for a standardized assessment of the number average molecular mass of the PHA-in-biomass. The standardized method is based on experience of extraction conditions (solvent-loading-time-temperature) where molecular weight loss is generally experienced to be negligible (WO2012/022998A1). However, such extraction conditions are generally with very low volumetric productivity and so economically prohibitive at industrial scale.

In the PHA accumulation process, one typically strives for the PHA-in-biomass weight average molecular mass to be in excess of 500 kDa but preferably in excess of 600 kDa and more preferably higher than 1000 kDa. It is generally desirable to obtain a polymer after extraction with a weight average molecular mass in excess of 350 kDa, preferably in excess of 400 kDa, more preferably in excess of 500 kDa and most preferably in excess of 700 kDa.

Notwithstanding a general benefit of producing a PHA of as high a molecular weight of possible, the criteria for molecular weight will be related to the material intent in application. Thus, the practical objective for the extraction process in practice is to simply meet preferred quality goals for the recovered polymer in relation to its intent of use. It makes no sense to recover a polymer in far excess of those goals to the expense of a less economically viable extraction process. The challenge is that for the extraction process to be more economically effective the selected conditions of loading-time-temperature and solvent must be such that some degree of molecular mass is allowed to be sacrificed in the course of the recovery process. Molecular weight loss is generally due to conditions that promote random-chain scission reactions to occur. The kinetics of random chain scission are influenced by the type of solvent, the solvent temperatures, and the PHA-rich-biomass loading in the solvent PHA-rich-biomass contains PHA and non-PHA biomass. It was observed (Example 7) that some (extractable and non-extractable) non-PHA biomass constituents contribute to random chain scission reactions.

Therefore, in selecting conditions of time and temperature for a particular solvent extraction, one must consider the combined effect of PHA-in-biomass loading and non-PHA biomass loading to the recovery process. In general, we have found that, all other things being equal, with increased loading of total biomass in the extraction process one may expect an increase in the scission rate.

The problem, as found in Example 7, is that not all non-PHA biomass will influence scission rate. Further, we experience that different PHA-rich biomass batches can exhibit different specific scission rates under similar extraction temperature and loading conditions, It may be hypothesized that the specific scission rate is governed by a rate constant and the concentrations of any reactive chemical compounds in solution. We have seen that a fraction of the non-PHA biomass may comprise such reactive chemical compounds. Therefore, assuming first order kinetics we felt that the scission rate could be described in general as being directly dependent on the biomass loading as follows:

$$\bar{r}_s = k_s \left( X_n + X_p \right) \qquad \text{EQ-E8.1}$$

where,

$r_s$ = the average specific scission rate
$k_s$ = scission rate constant dependent on temperature and biomass chemical composition;
$X_p$ = PHA-in-biomass loading per unit volume of extraction solvent;
$X_n$ = non-PHA biomass loading per unit volume of extraction solvent.

One of our objectives in developing these processes and methods has been to establish how best to tailor the biomass loading conditions for PHA extraction in PHA-poor solvents in order to:

1. Strive for the highest possible loading towards ensuring more economic volumetric productivity and generally improved gelation characteristics, while
2. Ensure at the same time that the selected loadings of PHA-in-biomass and non-PHA biomass yields an extracted polymer molecular weight equal to or above a specifiable threshold of product quality.

We sought to define the $k_s$ dependency on measurements reflecting the biomass chemical quality. To this end we examined the relationship between biomass loadings versus resultant scissions based on polymer extractions from a 1 L reactor with 2-butanol. PHA-rich-biomass from the same and distinctly different production batches were extracted with varying loading conditions ranging in PHA-loadings ($X_P$) from 25 to 80 g-PHA/L. In some cases the same PHA-rich biomass was extracted with or without pre-extraction using water or 2-butanol as the pre- extraction solvent. In these experiments, a nominal extraction time of 29 minutes at 138 °C was applied. The respective biomass qualities of "$X_n+X_P$" were quantitatively discriminated by TGA and FTIR (Example 1). PHA-in-biomass molecular weight was determined based on polymer characterization from standardized test tube scale extractions (Example 5),

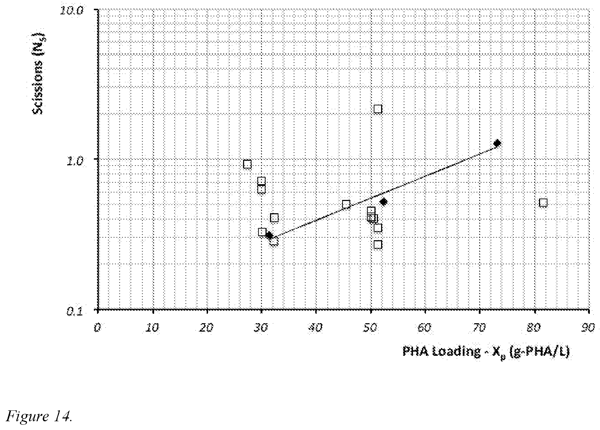

All the biomass samples for this particular example exhibited good PHA-in biomass thermal stability in keeping with the expectations of the methods applied according to WO2012/022998A1. However, a range of scission rates were observed and this suggested differences in the chemical stability of the PHA during the 1 L extraction process with 29 minutes at 138 °C (Figure 14 - Influence of PHA loading of PHA-rich-biomass on scissions for a given extraction time, temperature and solvent. A given biomass exhibits a trend of increased scissions with loading (♦). However, in general different biomass batches (□) exhibit wide variation of the polymer chemical stability during extraction). We felt that differences in the biomass chemical composition should be a principal factor to the respective observed differences in molecular mass loss rates.

The biomass chemical composition is captured as a "fingerprint" in an FTIR spectrum. The FTIR spectra were background corrected and the signals were normalized based on absorbances between 800 and 1800 nm, The spectra were then scaled by the mass "$X_n+X_p$" applied for each extraction. Thus, every batch of polymer extracted was accompanied by data of specific scission rate and an FTIR chemical "fingerprint" of the biomass loading.

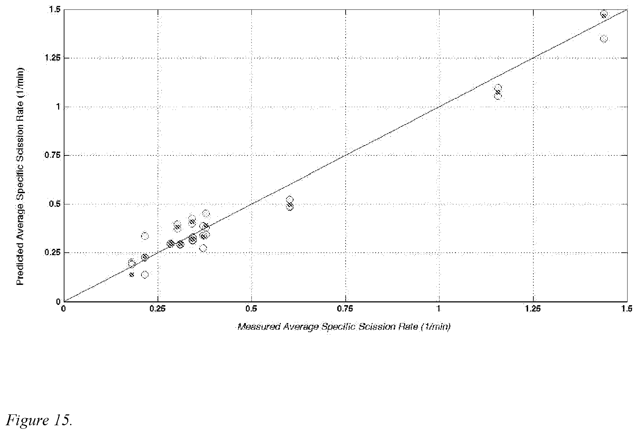

We discovered that these data could be used to formulate a model, in keeping to equation EQ-E8.1 and by partial least squares analysis, correlating the biomass fingerprint and loading to predict the respective average specific scission rates (Figure 15 - An illustration of PLS Model results (with model building experimental data (○) and model validation data (■)) relating the measured average specific scission rate to the predicted average specific scission rate based on an FTIR "fingerprint" of the biomass quality for distinct biomass batches with varying PHB-rich-biomass and loading conditions but with constant solvent type, average extraction temperature, and extraction time).

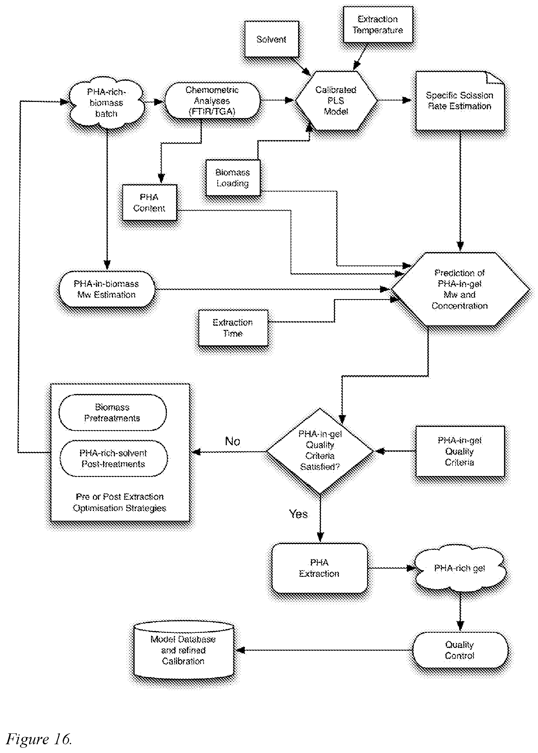

Without limitation, an example of how such a chemometric model is to be applied according to these disclosed findings is provided in Figure 16 (Example of PLS chemometric model application towards maintaining optimized extraction operating conditions for consistent process productivity and product quality levels with respect to incoming batch-to-batch varying PHA-rich-biomass quality). Those skilled in the art will understand that such models provide for an ability to assess biomass a priori to a calibrated extraction process and estimate the modelled specific scission rate for a particular biomass loading. An appropriate loading can be selected such that for a given initial molecular weight, and specific scission rate, the recovered PHA average molecular mass can with a high degree of certainty consistently meet a specified molecular weight standard.

## Example 9 - Material Flow and Process Management Example

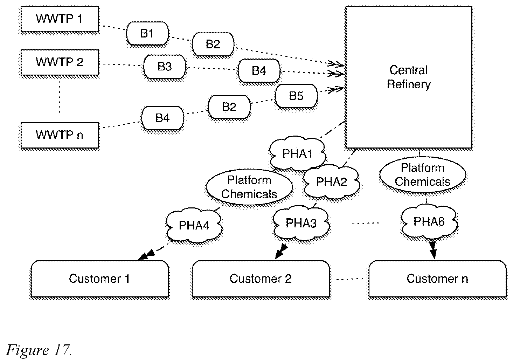

One may envision, in the implementation of the embodiments of PHA recovery presented herein, a centralized facility for receiving and processing PHA-rich biomass. Such a facility may be considered part of a refinery whereby incoming raw materials are refined to value-added materials and products (Figure 17 - A centralized refinery receiving batches of PHA-rich-biomass (B1 ..Bn of variable quality) from any number of wastewater treatment plants (WWTP1...WWTPn) and producing grades of biopolymer products (PHA1... PHAn) of consistent quality alongside other recovered value added chemicals for any number of commercial markets (Customer 1 .. Customer n)). The input batches of PHA-rich-biomass are anticipated to be of variable quality, including, but not limited to, PHA-in-biomass average molecular mass and chemical stability during extraction.

At least some embodiments of the present invention may be applied in order to accommodate feedstock batch-to-batch biomass quality variability, and in so doing consistently recover in a PHA-rich solvent gel, a polymer within established product or application criteria for the material average molecular mass.

The refinery achieves the constraints of the product quality criteria while at the same time working to be profitable by maximizing the volumetric productivity of the recovery process. As part of the recovery process non-PHA residuals (platform chemicals) may be recovered in parallel and the chemical and/or energy content of these platform chemicals may also be exploited. As examples, the recovery process may be further used to produce lipids, bio-oils, mineral nutrients (nitrogen and phosphorus), synthesis gas, and/or heat.

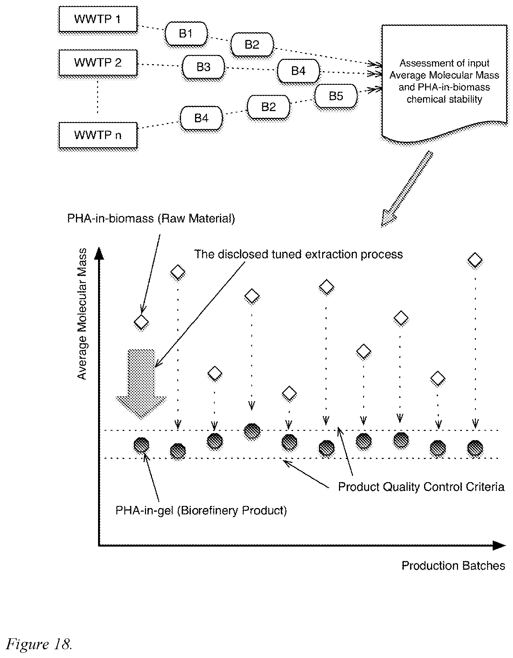

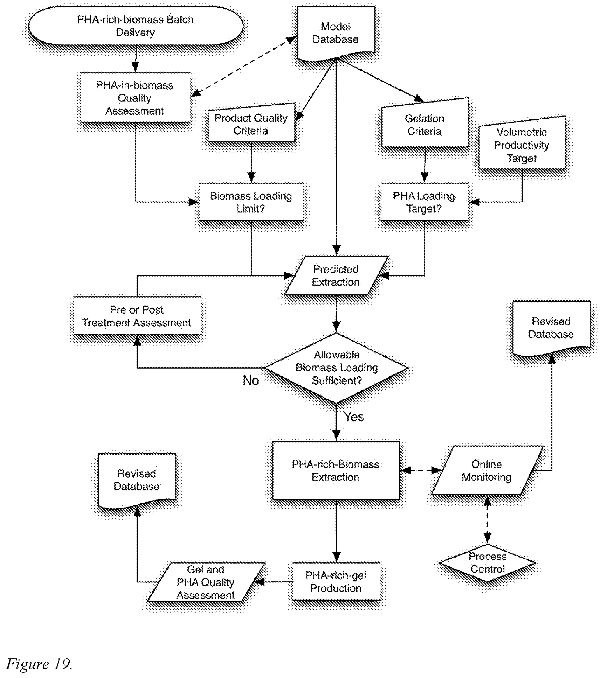

In one embodiment (Figure 18 - Application of the process and methods towards tuning the recovery process, and recovering a PHA of defined quality while applying the most aggressive process volumetric loading possible for overall improved economy and productivity. In general a high PHA-in-biomass average molecular mass allows for higher extraction loading with all other things being equal) the quality of input batches of PHA-rich-biomass are assessed with respect to molecular weight and chemical stability (Example 8). From these data the appropriate biomass loading to the disclosed extraction process may be estimated for producing a PHA in a gel or as a dried resin within quality control limits of consistent molecular mass. The disclosed methods of producing a PHA-rich-solvent-gel from a PHA-rich-biomass are directed towards maintaining a consistent product quality with optimum recovery process volumetric productivity. Batches of PHA-rich-biomass are in the first hand assessed (Figure 19 - Flow of material and information as part of applying methods of tuning process conditions in the conversion of a variable batch-to-batch quality of PHA-rich-biomass to a PHA-rich-solvent gel with consistently controlled quality). Such assessment provides information on the PHA content, PHA-in-biomass average molecular mass, PHA-in-biomass chemical stability, critical extraction temperatures and kinetics, and the extracted polymer critical gelation times and temperatures (Examples 1-5, 11). Based on the process model database and given information on the product quality criteria, an estimation of the maximum biomass loading can be made given solvent, time and temperature in the extraction (Example 8).

In parallel, an estimation of the requisite PHA-loading can be made in keeping with targets of gel properties and process volumetric productivity (Example 11). Given the PHA content of the biomass, the PHA-loading criteria can be compared to the allowable PHA-rich-biomass loading limit. If the allowable PHA-rich-biomass loading limit is not sufficient, then options of pre-treatment of the PHA-rich-biomass, or post treatment of the PHA-rich-solvent with chemical additives may be evaluated in order to gain an increase in the "allowable process biomass loading" (Examples 6, 7). With solvent-concentration-time-temperature conditions defined, the PHA may be extracted from the biomass with a-priori knowledge of the anticipated product quality. Online monitoring of the extraction process (Example 10) permits for fine-tuning of the extraction process time, and quality assessment of the gel and recovered PHA permits for feedback to the characterization and process-modelling database. Over time of feedback from assessment of the product quality, a revised model database permits for improved process tuning and more tightly controlled product quality management.

## Example 10 - PHA Extraction Process Online Monitoring

We have discovered a protocol to predict the kinetics of extraction of PHA from PHA-rich biomass based on experimental protocols at test tube scale (Example 3). Notwithstanding, a theoretical model with governing constants that are derived from laboratory tests may not predict more specifically all the factors that may influence mass transfer of PHA from a biomass into a solvent In a scaled up industrial process. The scaled up process control may nevertheless need to be tuned to achieve the best practically possible extraction kinetics on a case-by-case basis. Correction factors may be applicable in order to adjust a theoretical model to more closely represent practical experience in any particular implementation of the process industrialization in practice. Therefore, we found it to be advantageous to establish a means to monitor the progress of extraction through online process monitoring. Without limitation, two methods are illustrated herein in order to provide practical examples of measurement principles, which may be readily employed in scaled up extraction facilities.

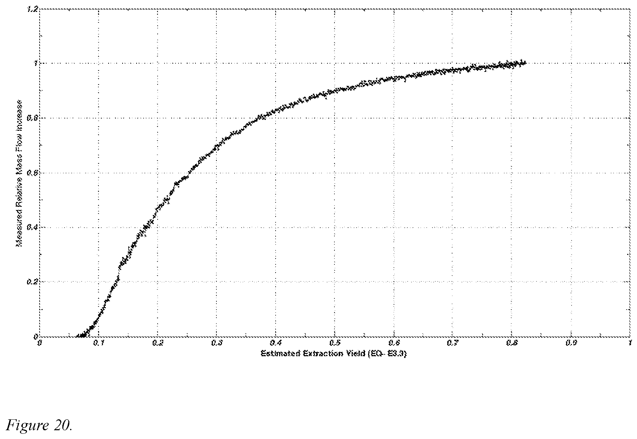

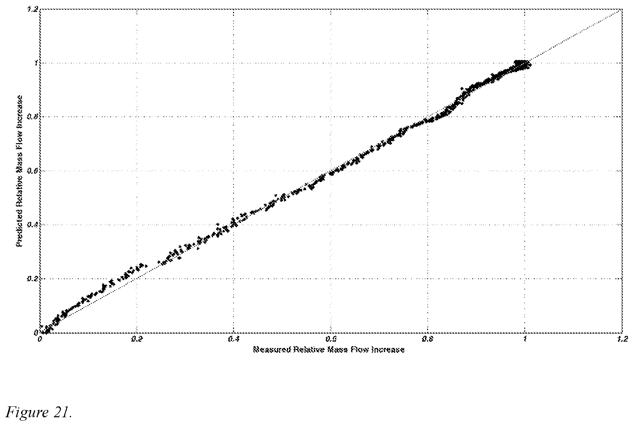

The extraction solvent viscosity will be a function of temperature but also a function of the concentration of polymer (and polymer molecular mass) in solution. Solvent viscosity decreases with temperature and increases with polymer concentration and/or molecular mass. We have found, through 10 L extraction experiments and mass flow on line monitoring and constant displacement pump frequency, that temperature-compensated solvent viscosity changes correlated to the anticipated kinetics for the PHA extraction (Figure 20 - Illustration of correlation between the relative change in measured solvent viscosity dependent recirculation flow (10 L process - Example 1) and the estimated modeled PHA extraction yield (Example 3)). In a second example, solvent NIR spectroscopic absorption is also dependent on temperature and the amount of polymer in solution. We have found that NIR absorption data can be used to monitor the progress of PHA extraction. The trends of changes in viscosity dependent recirculation mass flow due to PHA concentration during the extraction correlated with the measured trends of NIR absorption based on a PLS model of interpreting the logged spectra changes (Figure 21 - Illustration of a correlation based on a PLS model between the relative change in measured solvent viscosity dependent recirculation flow (10 L process - Example 1) and the predicted mass flow increase derived from spectra from online monitoring of the solvent by Near Infrared Spectroscopy (NIR)).

Without limitation, other monitoring parameters that we have found are of use in monitoring the polymer recovery include trends in reactor temperature and pressure. Those skilled in the art will understand that the online monitoring provides a powerful tool for the process operation and control, and that the predictive models presented herein may be refined based on the monitoring experience of extraction trends in practice,

**Example 11** - **Control and Exploitation of Gelation Kinetics**

Separation and recovery of the PHA-rich solvent from the biomass may be readily achieved before the solvent forms a gel. In the embodiments described herein, biomass containing PHA is placed into a pressure vessel together with a PHA-poor solvent such as 2-Butanol. The total amount of biomass combined with the solvent in the pressure vessel is adjusted so that the PHA loading is between 30-100 g PHA/L The temperature is increased on average to preferably above $T_{L15}$ and under $T_{U15}$ in the pressure vessel creating an overpressure, for 2-butanol, of 1 to 10 bars because 2-Butanol boils at 99 °C PHA extraction is then performed by mixing the solvent relative to the biomass at the extraction temperature(s) for longer than 15 minutes and less than 2 hours but preferably less than 1 hour. The PHA-rich solvent may then be separated from the residual biomass suspended solids by, for example, passing the PHA-rich solvent through suitably sized mesh screens.

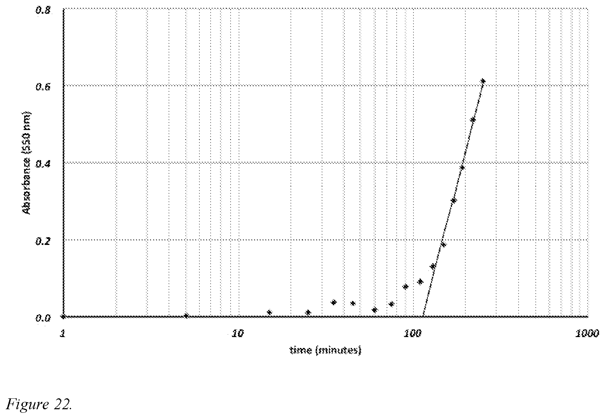

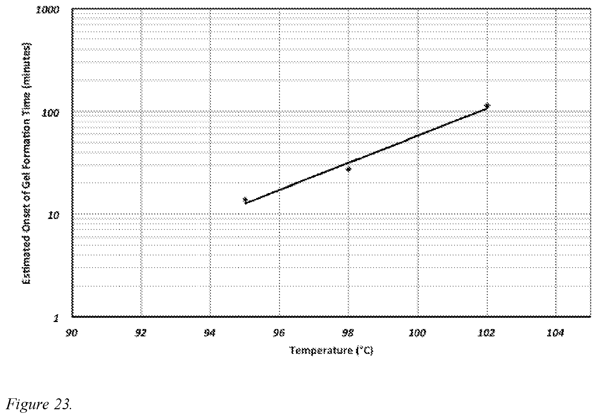

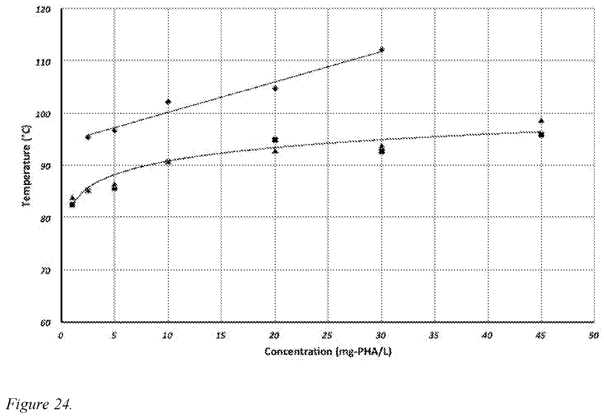

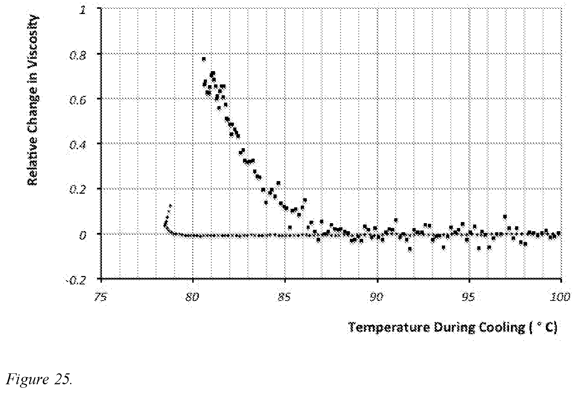

Before separation, the solvent can be cooled to below the extraction temperature of $T_{L15}$, but the time available for the processing task of separation will be influenced by the temperature dependent kinetics for the onset of gelation (Figure 22 - An illustration of the isothermal (102 °C) kinetics of PHA-rich-solvent gel formation kinetics based on increase of solvent optical density with gelation). We found that the gelation temperature shifts with polymer concentration, mixing conditions, and the type of co-polymer or co-polymer blend extracted (Figure 23 (An illustration of the influence of temperature on isothermal gelation time based on the onset of gelation illustrated in Figure 22); Figure 24 (An illustration of the influence of PHA-rich-solvent concentration and PHA composition ( ♦-PHB,▲ and ■ PHBV co-polymers) on gelation temperatures); and Figure 25 (An illustration of onset of gelation with the influence of high (♦ -onset below 78 °C) and low (■ - onset by 87 °C) mixing energies given similar cooling rates (≈ -1.6 ºC/min). Note that cooling rate was influenced by the exothermic nature of gelation)). Thus, the practical available processing times, mixing conditions, and temperatures during the residual biomass separation, and while the PHA-rich solvent is disposed to the location of gelation, may be adjusted as appropriate on a case-by-case batch-to-batch basis. Conditions are provided on established trends as a function of PHA concentration, temperature, or mixing.

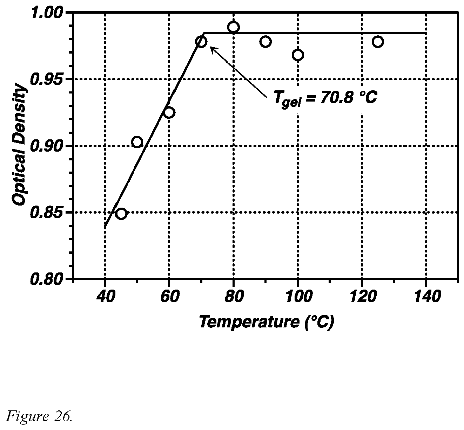

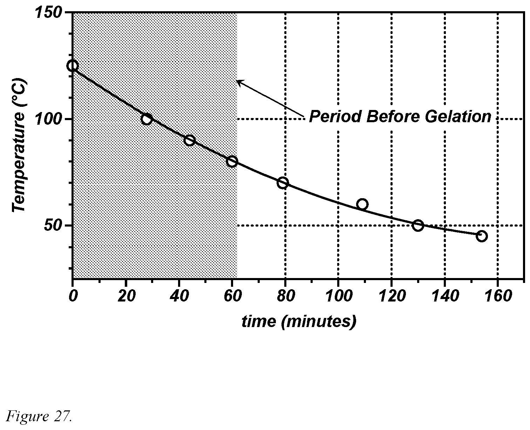

Without limitation, the time available for separating the biomass from the PHA-rich solvent and for disposing the solvent to the location of gelation can be evaluated on a batch-to-batch basis simply by a laboratory-scale experiment. PHA-rich biomass is loaded into a test tube together with an extraction solvent. The PHA in the biomass is extracted by heating the tube and contents in a suitable temperature controlled heater block, with periodic mixing, and above the determined $T_{L15}$ for the requisite extraction time. The biomass is allowed to settle in the tube and the contents of the tube are cooled in time in a manner that is representative of the full-scale system. At selected time intervals, the PHA-rich solvent supernatant optical density that is above the settled biomass is measured. Such measurements can be made in a spectrophotometer, but also more simply with a digital camera and a standard flash (Figure 26 - An illustration of onset of gelation as determined by regression analysis of the optical density as a function of temperature for a PHA-rich solvent). The trend in optical density for the PHA-rich solvent can be assessed by regression analysis and a gelation temperature can be identified. The cooling time before reaching the gelation point can then be estimated from the cooling curve (Figure 27 - An illustration the available time before onset of gelation for a given PHA-rich solvent cooling curve with reference Figure 26).

Once the PHA-rich solvent is separated from the suspended residual biomass, further cooling or reduction in mixing intensity will promote gel formation as the PHA-rich solvent is conveyed out of the extraction reactor. The gel can be pumped, extruded, or pressed. Solvent may be exuded from the gel mass by applying mechanical forces using any number of various available techniques including for example a filter press. The gel can also be processed by blending with other gels of PHA and or blended with chemical additives before or as part of applying mechanical forces to exude excess solvent from the matrix.

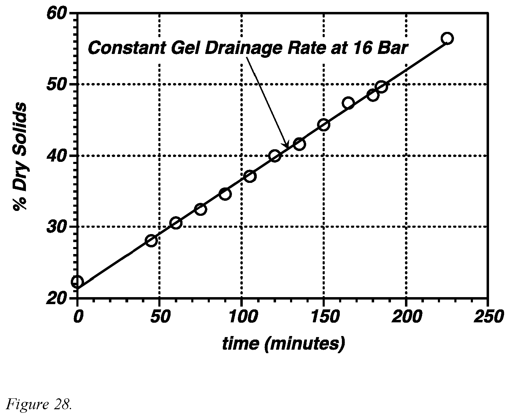

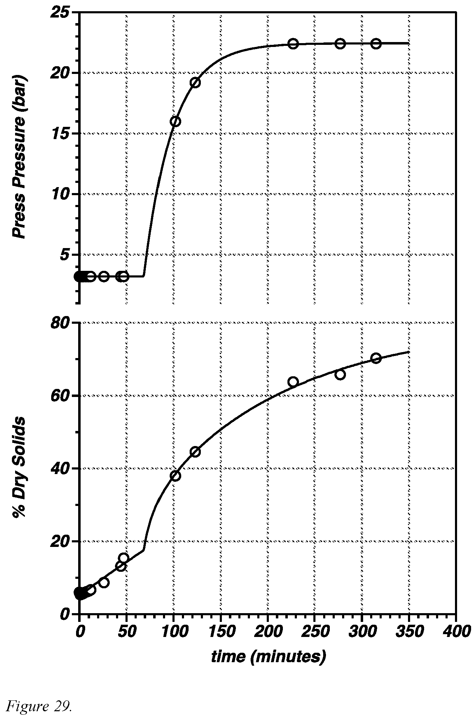

Generally, the pressure applied to the gel matrix influences the amount of residual solvent expressed. For a given pressure, solvent is expressed at a rate that can be strongly influenced by the cake geometry, the type of press used, and the resulting resistance of solvent drainage. An example for a particular press is presented in Figure 28 (Influence of drainage rate at constant pressure (16 bar) on solvent expression and increase of gel dry solids content from a PHA-rich-gel). For a given final press pressure and particular press, an ultimate level of dry solids may be achieved (Figure 29 - Influence of time and pressure on solvent expression from a PHA-rich-gel) and we find that these levels are also related to the type of PHA.

Notwithstanding, we have observed with replicate pressing trials of recovered PHBV from mixed culture PHA-rich biomass that pressing pressures between 15 and 25 bar can yield resultant pressed cake dry solids content generally between 55 and 75 percent, and generally a polymer purity of greater than 99 percent.

The expressed solvent can be collected and recycled. As part of the solvent recovery, chemical by-products such as lipids and fatty acids can be co-recovered. The residual solvent in the concentrated gel can be removed through thermal processing with or without further solvent rinsing for extended degree of the polymer purification.

### Example 12 - Process Examples

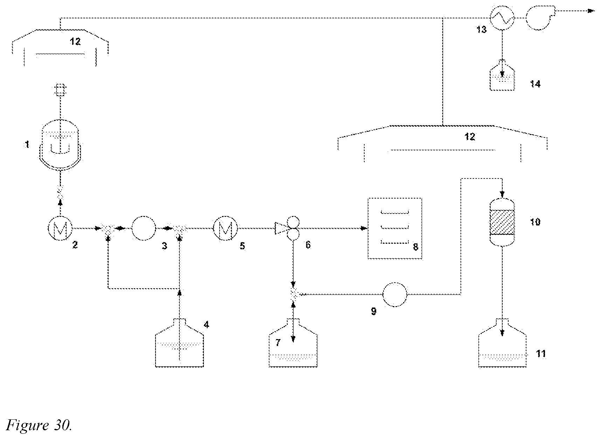

Without limitation as illustrated in Figure 30 (A schematic illustration of elements of the extraction process), a batch extraction reactor (1) is filled with clean solvent by pumping (3) from a solvent reservoir (4). The reactor (1) is charged with biomass, sealed, and pressurized, and PHA is extracted into the solvent at temperatures in excess of $T_{L15}$. Extraction time is established through predictive modelling and/or by monitoring of the extraction process using on-line sensing of the solvent based on viscosity, light absorption, or other analogous techniques to determine the evolving process trend of increasing PHA concentration. After the extraction phase, the solvent is discharged (3) out of the reactor (1) through a heated line (2) where the requisite heating level is established so as to permit a practical transfer timeframe where gelation onset is avoided during the residual biomass separation. Residual suspended biomass is separated by filtration in the reactor or in a trap located downstream of the reactor in the solvent exit line. PHA-rich solvent is conveyed through a heat exchanger (5) that promotes sufficient cooling for gel formation and the gel is mechanically pressed with pressures between 5 and 30 bar (6). Expressed spent solvent is collected (7) and the PHA cake is dried by thermal methods (8). In another embodiment, the gel is not dried but is used directly in further processing and blending in gel form.

Fresh solvent from (4) can be used to flush the remaining PHA gel from the system.

Spent extraction solvent (7) is pumped (9) and recovered by evaporation (10) and recycled solvent (11) is returned to the solvent reservoir (4). Non-PHA extracted biomass constituents such as lipids and fatty acids can be recovered and also valorised. Solvent evaporation or fugitive emissions are captured (12), condensed (13), and recovered (14). Biomass residual that was captured by physical separation during removal from (1) is further processed for chemical and energy recovery such as through pyrolysis. The phosphorus containing ash content as a result of the pyrolysis can be further used as a by-product raw material for applications such as in agriculture.

Variations in configuration or enhancement of the above process and method, or modes of its industrial scale implementation, may be considered. In one embodiment, the biomass is pre-extracted with a solvent below $T_{L15}$ in order to improve the PHA-in-biomass chemical stability. In another embodiment, the PHA-rich solvent disposed from physical separation from the biomass, is combined with chemical additives before, during or after gelation. In a further embodiment, the heated solvent is re-circulated through the biomass rather than mixing the biomass in the heated solvent. In a fourth embodiment, the biomass is loaded in an external chamber and the PHA-poor solvent is pre-heated before being brought in contact with the biomass. The heated solvent is recirculated through the biomass-containing chamber (in upflow or downflow). The biomass is retained in said chamber. In a fifth embodiment, a multitude of batch extraction reactors are configured to share a common infrastructure of gelation and solvent-gel separation.

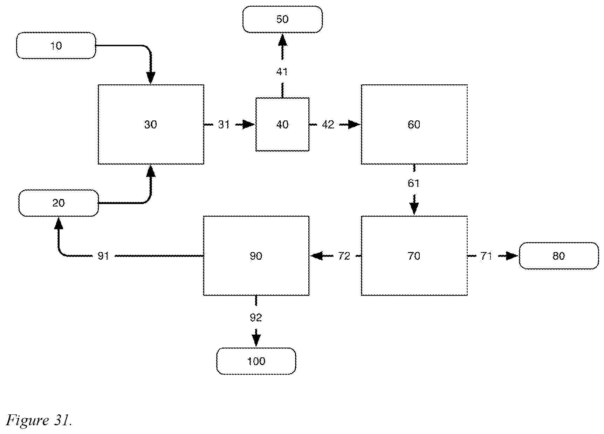

The general PHA extraction process material flow is summarized in Figure 31 (A schematic illustration of the material flow in the extraction process). A selected mass of granulated biomass (10) with a selected volume of PHA-poor solvent (20) are combined in an extraction system (30). The extraction system may include on-line sensors that enable the monitoring of the progress of extraction in time. In extraction system (30), elevated temperatures above $T_{L15}$ are applied for a determined time whereby the mix of PHA-rich solvent and biomass granulate (31) can be separated (40) to a residual spent granulate (41) and a PHA-rich solvent (42). The spent granulate can be collected (50) and this material may be further processed to produce energy and chemicals (50). PHA-rich solvent is disposed to a location of gelation (60) and the gelated material is disposed (61) such that it can be mechanically engaged (70). From 70, PHA is recovered (71) and the PHA may be collected (60) and further processed. Selected chemical additives and/or other gelated PHA batches may be combined at 42, 60, 61 , 70, and/or 71 in order to manipulate the polymer properties or in anticipation of providing compounding additives towards the formulation of a bioplastic. Spent solvent (72) recovered from mechanically engaging the gel (70) is conveyed to a solvent recovery process (90), from which residues (92) are collected (100). These residues that include lipids and fatty acids may be further processed and valorised. Purified solvent (91) is recovered and reutilised (20) in subsequent batch extraction runs.

**Claims**

1.  A method of recovering polyhydroxyalkanoates (PHA) from mixed culture PHA-containing biomass, comprising:

    - determining an isothermal extraction trend as a function of temperature;
    - empirically modelling the isothermal extraction trend to a sigmoid function ($f_{15}$) that describes the trend of increased extent of extracted polymer with temperature after 15 minutes according to:

    $$f_{15} = \frac{1}{1 + e^{-k_{15}(T-T_H)}}$$

    where

    $f_{15}$ = fraction of extractable polymer after 15 minutes as a function of Temperature (T);
    $k_{15}$ = 15 minute extraction rate temperature coefficient;
    $T_H$ = the temperature for $f_{15}$ equal to 0.50;

    - determining, from the sigmoid function ($f_{15}$), a lower limiting temperature $TL_{15}$ and an upper limiting temperature $TU_{15}$, wherein the lower limiting temperature $TL_{15}$ is a temperature below which negligible extraction will occur within 15 minutes and the upper limiting temperature $TU_{15}$ is a temperature where significant extraction will occur within 15 minutes;
    - directing the biomass into a reactor;
    - mixing a solvent with the biomass in the reactor;
    - heating the solvent and the biomass in the reactor;
    - extracting the PHA from the PHA-containing biomass by dissolving the PHA in the solvent to form a PHA-rich solvent;
    - maintaining the PHA solvent at a temperature between the lower limiting temperature $TL_{15}$ and the upper limiting temperature $T_{U15}$;
    - transferring the PHA-rich solvent from the reactor to a PHA separator where the PHA is separated from the PHA-rich solvent;
    - while transferring the PHA-rich solvent to the PHA separator, maintaining the PHA-rich solvent at a temperature that prevents the PHA-rich solvent from assuming a gelation state;
    - cooling the PHA-rich solvent to form a PHA-rich solvent gel; and
    - mechanically pressing the PHA-rich solvent gel at the PHA separator location and causing the solvent to be removed from the PHA-rich solvent gel.

2.  The method of claim 1, wherein prior to directing the biomass into the reactor, grinding the biomass to form biomass granules and after grinding the biomass to form the biomass granules, directing the biomass granules into the reactor and contacting the biomass granules with the solvent.

3.  The method of claim 1, wherein the PHA in the biomass directed to the reactor is thermally stable and wherein the PHA in the biomass includes a thermal decomposition temperature greater than 270°C.

4.  The method of claim 1, including directing the PHA-rich solvent through a heat exchanger disposed in a first section of a conduit and heating the PHA-rich solvent by transferring heat from the heat exchanger to the PHA-rich solvent and/or including directing the PHA-rich solvent through a heat exchanger in a second section of the conduit and then cooled by transferring heat between the heat exchanger in the second section of the conduit and the PHA-rich solvent.

**5.** The method of claim 1, including mechanically pressing the PHA-rich solvent gel to expel spent solvent from the PHA-rich solvent gel and purifying the spent solvent by subjecting the spent solvent to an evaporation process and producing a solvent vapour and the method further includes condensing the solvent vapour to form a purified solvent.

**6.** The method of claim 5, including recovering chemical products such as lipids and fatty acids as part of the purification and recovery of the extraction solvent.

**7.** The method of claim 1, including separating the biomass from the PHA-rich solvent and subjecting the separated biomass to an incineration or pyrolysis process, wherein the method preferably further includes recovering phosphorus from the residual material after incineration or pyrolysis.

**8.** The method of claim 1, wherein the sigmoid function $f_{15}$ comprises a series of sigmoid functions with distinctly different regions of homogeneously extractable fractions, according to:

$$\frac{df_i}{dt} = \sum_{i=1}^{n}\frac{df_i}{dt} = \sum_{i=1}^{n} k_{ei}(a_i - f_i), \sum_{i=1}^{n} a_i = 1, f_i \le a_i$$

where:

$f_i$ = the $i^{th}$ fraction of extracted PHA (0 < fi < ai);
$k_{ei}$ = the $i^{th}$ fraction's 1st order extraction rate constant.

**9.** The method of claim 1, including:

- mixing the biomass with a PHA-poor solvent in the reactor;
- prior to introducing the biomass into the reactor, forming the biomass into granules with the granules having a particle size distribution with D50 between 0.1 and 4 mm;

wherein the PHA in the biomass directed to the reactor is thermally stable and includes a thermal decomposition temperature greater than 270°C; and

- after extracting PHA from the PHA-containing biomass, separating the PHA-rich solvent from the biomass and maintaining the temperature of the PHA solvent sufficient to prevent gelation during the separation process.

**10.** Method of claim 1, wherein during the step of extracting PHA from the PHA-containing biomass providing an online monitoring process for monitoring kinetics and progress of PHA extraction, wherein the online monitoring process is employed to control the extraction time for obtaining a PHA-rich solution.

**11.** Method of any preceding claim, wherein the method comprises real-time fine-tuning the extraction time from batch to batch, wherein the fine-tuning preferably comprises fine-tuning of the extraction process time based on online monitoring of the extraction process.

**12.** The method of claim 10 wherein the online monitoring process comprises measuring changes in the viscosity of the PHA-poor solvent over a selected time period, and wherein the measuring preferably comprises measuring temperature compensated changes in the viscosity of the PHA-poor solvent over a selected time period.

**13.** Method of claim 10, wherein the online monitoring process comprises monitoring the PHA-poor solvent by near infrared spectroscopy (NIR) or comprises monitoring the trends of pressure as a function of temperature in an extraction vessel.

**14.** Method of claim 1, comprising selecting a biomass loading to the extraction system based on a bench scale trial to determine the specific scission rate as described in the description, so that the recovered PHA weight average molecular mass is at least 350 kDa, preferably greater than 500 kDa, more preferably greater than 700 kDa.

**15.** A method of recovering PHA from a mixed culture biomass comprising:

a. preparing a granulated PHA-rich biomass;

b. determining an isothermal extraction trend as a function of temperature;

c. empirically modelling the isothermal extraction trend to a sigmoid function (f15) that describes the trend of increased extent of extracted polymer with temperature after 15 minutes according to:

$$f_{15} = \frac{1}{1 + e^{-k_{15}(T - T_H)}}$$

where

$f_{15}$ = fraction of extractable polymer after 15 minutes as a function of Temperature (T);

$k_{15}$ = 15 minute extraction rate temperature coefficient;

$T_H$ = the temperature for $f_{15}$ equal to 0.50;

d. determining, from the sigmoid function ($f_{15}$), a lower limiting temperature $TL_{15}$ and an upper limiting temperature $TU_{15}$, wherein the lower limiting temperature $TL_{15}$ is a temperature below which negligible extraction will occur within 15 minutes and the upper limiting temperature $TU_{15}$ is a temperature where significant extraction will occur within 15 minutes;

e. admixing the granulated PHA-rich biomass and a PHA-poor solvent;

f. extracting the PHA from the PHA-rich biomass to produce a PHA-rich solvent by dissolving the PHA in a PHA-poor solvent at temperatures above a limiting extraction temperature ($TL_{15}$);

g. during extraction, maintaining the temperature of the PHA-poor solvent such that the temperature, on average, is between $TL_{15}$ and $TU_{15}$ for a period of time greater than 15 minutes, preferably less than 1 hour and most preferably less than 2 hours;

h. after extracting PHA from the PHA-rich biomass, separating the PHA-rich solvent from the residual biomass while maintaining the temperature above a gelation temperature ($T_{gel}$);

i. transferring the PHA-rich solvent to a location of gelation while maintaining the temperature of the PHA-rich solvent temperature ($T_{gel}$) at a temperature that prevents gelation;

j. promoting PHA-rich solvent gelation in a predetermined location in the process by cooling the PHA-rich solvent to a temperature at or below the gelation temperature with or without mixing; and

k. pressing the solvent away from the PHA-rich solvent gel.

**Patentansprüche**

**1.** Ein Verfahren zur Gewinnung von Polyhydroxyalkanoaten (PHA) aus PHA-haltiger Biomasse in Mischkultur, aufweisend die folgenden Schritte:

- Bestimmen eines isothermen Extraktionsverlaufs als Funktion der Temperatur;

- empirisches Modellieren des isothermen Extraktionsverlaufs durch eine Sigmoidfunktion ($f_{15}$), die nach 15 Minuten den Anstieg des Anteils des extrahierten Polymers mit der Temperatur beschreibt, gemäß der Beziehung:

$$f_{15} = \frac{1}{1 + e^{-k_{15}(T - T_H)}}$$

wobei gilt:

$f_{15}$ = Anteil des extrahierbaren Polymers nach 15 Minuten als Funktion der Temperatur (T);

$k_{15}$ = Temperaturkoeffizient der Extraktionsrate nach 15 Minuten;

$T_H$ = die Temperatur für $f_{15}$ gleich 0,50;

- Bestimmen einer unteren Grenztemperatur $TL_{15}$ und einer oberen Grenztemperatur $TU_{15}$ aus der Sigmoidfunktion ($f_{15}$), wobei die untere Grenztemperatur $TL_{15}$ eine Temperatur darstellt, unter der innerhalb von 15 Minuten eine vernachlässigbare Extraktion stattfindet, und die obere Grenztemperatur $TU_{15}$ eine Temperatur darstellt, bei der innerhalb von 15 Minuten eine signifikante Extraktion stattfindet;

- Einleiten der Biomasse in einen Reaktor;
- Mischen eines Lösungsmittels mit der Biomasse im Reaktor;
- Erhitzen des Lösungsmittels und der Biomasse in dem Reaktor;
- Extrahieren des PHA aus der PHA-haltigen Biomasse durch Auflösen des PHA in dem Lösungsmittel, um ein PHA-reiches Lösungsmittel zu bilden;
- Halten des PHA-Lösungsmittels bei einer Temperatur zwischen der unteren Grenztemperatur $TL_{15}$ und der oberen Grenztemperatur $TU_{15}$;
- Überführen des PHA-reichen Lösungsmittels aus dem Reaktor in einen PHA-Separator, wo das PHA von dem PHA-reichen Lösungsmittel abgetrennt wird;
- Halten des PHA-reichen Lösungsmittels auf einer Temperatur, die verhindert, dass, während das PHA-reiche Lösungsmittel in den PHA-Separator überführt wird, das PHA-reiche Lösungsmittel einen Gelierungszustand annimmt;
- Abkühlen des PHA-reichen Lösungsmittels, um ein PHA-reiches Lösungsmittelgel zu bilden; und
- mechanisches Pressen des PHA-reichen Lösungsmittelgels am Ort des PHA-Separators und Veranlassen, dass das Lösungsmittel aus dem PHA-reichen Lösungsmittelgel entfernt wird.

2. Das Verfahren nach Anspruch 1, wobei, vor dem Einleiten der Biomasse in den Reaktor, die Biomasse gemahlen wird, um Biomasse-Granulat zu bilden, und nach dem Mahlen der Biomasse, um das Biomasse-Granulat zu bilden, das Biomasse-Granulat in den Reaktor geleitet wird und das Biomasse-Granulat mit dem Lösungsmittel in Kontakt gebracht wird.

3. Das Verfahren nach Anspruch 1, wobei das PHA in der dem Reaktor zugeführten Biomasse thermisch stabil ist, und wobei das PHA in der Biomasse eine thermische Zersetzungstemperatur von mehr als 270°C aufweist.

4. Das Verfahren nach Anspruch 1, aufweisend das Leiten des PHA-reichen Lösungsmittels durch einen in einem ersten Abschnitt einer Leitung angeordneten Wärmetauscher, und das Erhitzen des PHA-reichen Lösungsmittels durch Übertragung von Wärme vom Wärmetauscher auf das PHA-reiche Lösungsmittel und/oder aufweisend das Leiten des PHA-reichen Lösungsmittels durch einen Wärmetauscher in einem zweiten Abschnitt der Leitung und das anschließende Kühlen durch Übertragung von Wärme zwischen dem Wärmetauscher im zweiten Abschnitt der Leitung und dem PHA-reichen Lösungsmittel.

5. Das Verfahren nach Anspruch 1, bei dem das PHA-reiche Lösungsmittelgel mechanisch gepresst wird, um verbrauchtes Lösungsmittel aus dem PHA-reichen Lösungsmittelgel auszutreiben, und das verbrauchte Lösungsmittel gereinigt wird, indem das verbrauchte Lösungsmittel einem Verdampfungsprozess unterzogen wird und ein Lösungsmitteldampf erzeugt wird, wobei das Verfahren ferner das Kondensieren des Lösungsmitteldampfes zur Bildung eines gereinigten Lösungsmittels umfasst.

6. Das Verfahren nach Anspruch 5, einschließlich der Rückgewinnung von chemischen Produkten wie Lipiden und Fettsäuren als Teil der Reinigung und Rückgewinnung des Extraktionslösungsmittels.

7. Das Verfahren nach Anspruch 1, bei dem die Biomasse von dem PHA-reichen Lösungsmittel abgetrennt und die abgetrennte Biomasse einem Verbrennungs- oder Pyrolyseprozess unterzogen wird, wobei das Verfahren vorzugsweise ferner die Rückgewinnung von Phosphor aus dem Restmaterial nach der Verbrennung oder der Pyrolyse umfasst.

8. Das Verfahren nach Anspruch 1, wobei die Sigmoidfunktion $f_{15}$ eine Reihe von Sigmoidfunktionen umfasst, mit deutlich unterschiedlichen Bereichen homogen extrahierbarer Fraktionen, gemäß der Beziehung:

$$\frac{df_i}{dt} = \sum_{i=1}^{n} \frac{df_i}{dt} = \sum_{i=1}^{n} k_{ei}(a_i - f_i), \sum_{i=1}^{n} a_i = 1, f_i \leq a_i$$

wobei gilt:

$f_i$ = die i-te Fraktion des extrahierten PHA ($0 < f_i < a_i$);
$k_{ei}$ = die Extraktionsratenkonstante 1. Ordnung der i-ten Fraktion.

9. Das Verfahren nach Anspruch 1, einschließlich:

   - Mischen der Biomasse mit einem PHA-armen Lösungsmittel in dem Reaktor;
   - vor dem Einbringen der Biomasse in den Reaktor, Ausbilden der Biomasse zu Granulat, wobei das Granulat eine D50 Teilchengrößenverteilung zwischen 0,1 und 4 mm aufweist;

   wobei das PHA in der dem Reaktor zugeführten Biomasse thermisch stabil ist und eine thermische Zersetzungstemperatur von mehr als 270°C aufweist; und wobei

   - nach der Extraktion von PHA aus der PHA-enthaltenden Biomasse das PHA-reiche Lösungsmittel von der Biomasse abgetrennt wird und die Temperatur des PHA-Lösungsmittels ausreichend gehalten wird, um während des Trennungsprozesses eine Gelierung zu verhindern.

10. Das Verfahren nach Anspruch 1, wobei während des Schritts der Extraktion von PHA aus der PHA-haltigen Biomasse zur Überwachung der Kinetik und des Fortschritts der PHA-Extraktion ein Online-Überwachungsverfahren vorgesehen ist, wobei das Online-Überwachungsverfahren eingesetzt wird, um im Rahmen der Gewinnung einer PHA-reichen Lösung die Extraktionszeit zu steuern.

11. Das Verfahren nach einem der vorherigen Ansprüche, wobei das Verfahren eine Echtzeit-Feinabstimmung der Extraktionszeit von Charge zu Charge umfasst, wobei die Feinabstimmung vorzugsweise eine Feinabstimmung der Extraktionsprozesszeit auf der Grundlage einer Online-Überwachung des Extraktionsprozesses umfasst.

12. Das Verfahren nach Anspruch 10, wobei das Online-Überwachungsverfahren das Messen von Änderungen der Viskosität des PHA-armen Lösungsmittels über eine ausgewählte Zeitspanne umfasst, und wobei das Messen vorzugsweise das Messen von temperaturkompensierten Änderungen der Viskosität des PHA-armen Lösungsmittels über eine ausgewählte Zeitspanne umfasst.

13. Das Verfahren nach Anspruch 10, wobei das Online-Überwachungsverfahren die Überwachung des PHA-armen Lösungsmittels durch Nahinfrarot-Spektroskopie (NIR) oder die Überwachung der Druckveränderungen als Funktion der Temperatur in einem Extraktionsbehälter umfasst.

14. Das Verfahren nach Anspruch 1, aufweisend das Auswählen einer Biomassebeladung für das Extraktionssystem auf der Grundlage eines Versuchs im Labormaßstab zur Bestimmung der spezifischen Spaltrate, wie in der Beschreibung dargelegt, so dass die gewichtsgemittelte Molekülmasse des zurückgewonnenen PHA mindestens 350 kDa, vorzugsweise mehr als 500 kDa und noch bevorzugter mehr als 700 kDa beträgt.

15. Ein Verfahren zur PHA-Gewinnung aus einer Biomasse in Mischkultur, aufweisend die folgenden Schritte:

   a. Erzeugen einer granulierten PHA-reichen Biomasse;
   b. Bestimmen eines isothermen Extraktionsverlaufs als Funktion der Temperatur;
   c. empirisches Modellieren des isothermen Extraktionsverlaufs durch eine Sigmoidfunktion ($f_{15}$), die nach 15 Minuten den Anstieg des Anteils an extrahiertem Polymer mit der Temperatur beschreibt, gemäß der Beziehung:

$$f_{15} = \frac{1}{1 + e^{-k_{15}(T - T_H)}}$$

   wobei gilt:

   $f_{15}$ = Anteil des extrahierbaren Polymers nach 15 Minuten als Funktion der Temperatur (T);
   $k_{15}$ = Temperaturkoeffizient der Extraktionsrate nach 15 Minuten;
   $T_H$ = die Temperatur für $f_{15}$ gleich 0,50;

   d. Bestimmen einer unteren Grenztemperatur $TL_{15}$ und einer oberen Grenztemperatur $TU_{15}$ aus der Sigmoidfunktion ($f_{15}$), wobei die untere Grenztemperatur $TL_{15}$ eine Temperatur darstellt, unterhalb der innerhalb von 15 Minuten eine vernachlässigbare Extraktion stattfindet, und die obere Grenztemperatur $TU_{15}$ eine Temperatur darstellt, bei der innerhalb von 15 Minuten eine signifikante Extraktion stattfindet;
   e. Zumischen eines PHA-armen Lösungsmittels zu der granulierten PHA-reichen Biomasse;

f. Extrahieren des PHA aus der PHA-reichen Biomasse zur Herstellung eines PHA-reichen Lösungsmittels durch Auflösen des PHA in einem PHA-armen Lösungsmittel bei Temperaturen oberhalb einer Extraktions-Grenztemperatur (TL$_{15}$);

g. während des Extrahierens, Aufrechterhalten der Temperatur des PHA-armen Lösungsmittels, so dass die durchschnittliche Temperatur für eine Zeitspanne von mehr als 15 Minuten, vorzugsweise weniger als 1 Stunde und am meisten bevorzugt weniger als 2 Stunden zwischen TL$_{15}$ und TU$_{15}$ gehalten wird;

h. nach dem Extrahieren von PHA aus der PHA-reichen Biomasse, Trennen des PHA-reichen Lösungsmittels von der restlichen Biomasse, während die Temperatur über einer Gelierungstemperatur (T$_{gel}$) gehalten wird;

i. Überführen des PHA-reichen Lösungsmittels an einen Ort der Gelierung, während die Temperatur des PHA-reichen Lösungsmittels (T$_{gel}$) auf einer eine Gelierung verhindernde Temperatur gehalten wird,

j. Fördern der Gelierung des PHA-reichen Lösungsmittels an einer vorbestimmten Stelle des Verfahrens durch Abkühlen des PHA-reichen Lösungsmittels auf eine Temperatur bei oder unter der Gelierungstemperatur mit oder ohne Mischen; und

k. Abpressen des Lösungsmittels aus dem PHA-reichen Lösungsmittelgel.

## Revendications

1. Procédé de récupération de polyhydroxyalcanoates (PHA) à partir d'une biomasse contenant des PHA de culture mélangée, comprenant :

   - la détermination d'une tendance d'extraction isotherme en fonction de la température ;
   - la modélisation empirique de la tendance d'extraction isotherme en une fonction sigmoïde (f$_{15}$) qui décrit la tendance de l'augmentation du niveau de polymère extrait avec la température après 15 minutes conformément à :

$$f_{15} = \frac{1}{1 + e^{-k_{15}(T - T_H)}}$$

   où

      f$_{15}$ = fraction de polymère extractible après 15 minutes en fonction de la température (T) ;
      k$_{15}$ = coefficient de température de vitesse d'extraction de 15 minutes ;
      T$_H$ = la température pour f$_{15}$ égale à 0,50 ;

   - la détermination, à partir de la fonction sigmoïde (f$_{15}$), d'une température limitante inférieure TL$_{15}$ et d'une température limitante supérieure TU$_{15}$, dans lequel la température limitante inférieure TL$_{15}$ est une température en dessous de laquelle une extraction négligeable se produira en 15 minutes et la température limitante supérieure TU$_{15}$ est une température à laquelle une extraction importante se produira en 15 minutes ;
   - l'acheminement de la biomasse dans un réacteur ;
   - le mélange d'un solvant avec la biomasse dans le réacteur ;
   - le chauffage du solvant et de la biomasse dans le réacteur ;
   - l'extraction du PHA à partir de la biomasse contenant des PHA par la dissolution du PHA dans le solvant pour former un solvant riche en PHA ;
   - le maintien du solvant pour PHA à une température entre la température limitante inférieure TL$_{15}$ et la température limitante supérieure TU$_{15}$ ;
   - le transfert du solvant riche en PHA à partir du réacteur vers un séparateur de PHA où le PHA est séparé du solvant riche en PHA ;
   - tout en transférant le solvant riche en PHA vers le séparateur de PHA, le maintien du solvant riche en PHA à une température qui empêche le solvant riche en PHA de passer à un état gélifié ;
   - le refroidissement du solvant riche en PHA pour former un gel de solvant riche en PHA ; et
   - le pressage mécanique du gel de solvant riche en PHA à l'emplacement du séparateur de PHA et l'élimination du solvant à partir du gel de solvant riche en PHA.

2. Procédé selon la revendication 1, dans lequel, avant d'acheminer la biomasse dans le réacteur, le broyage de la biomasse pour former des granulés de biomasse et, après le broyage de la biomasse pour former des granulés de biomasse, l'acheminement des granulés de biomasse dans le réacteur et la mise en contact des granulés de biomasse avec le solvant.

3. Procédé selon la revendication 1, dans lequel le PHA dans la biomasse acheminée vers le réacteur est thermiquement stable et dans lequel le PHA dans la biomasse comporte une température de décomposition thermique supérieure à 270 °C.

4. Procédé selon la revendication 1, comportant l'acheminement du solvant riche en PHA à travers un échangeur de chaleur disposé dans une première section d'un conduit et le chauffage du solvant riche en PHA par transfert de chaleur à partir de l'échangeur de chaleur vers le solvant riche en PHA et/ou comportant l'acheminement du solvant riche en PHA à travers un échangeur de chaleur dans une deuxième section du conduit et ensuite un refroidissement par transfert de chaleur entre l'échangeur de chaleur dans la deuxième section du conduit et le solvant riche en PHA.

5. Procédé selon la revendication 1, comportant le pressage mécanique du gel de solvant riche en PHA pour expulser le solvant usé à partir du gel de solvant riche en PHA et la purification du solvant usé par soumission du solvant usé à un procédé d'évaporation et la production d'une vapeur de solvant et le procédé comporte en outre la condensation de la vapeur de solvant pour former un solvant purifié.

6. Procédé selon la revendication 5, comportant la récupération de produits chimiques, tels que des lipides et des acides gras, dans le cadre de la purification et de la récupération du solvant d'extraction.

7. Procédé selon la revendication 1, comportant la séparation de la biomasse du solvant riche en PHA et la soumission de la biomasse séparée à un procédé d'incinération ou de pyrolyse, dans lequel le procédé comporte en outre de préférence la récupération du phosphore à partir de la matière résiduelle après l'incinération ou la pyrolyse.

8. Procédé selon la revendication 1, dans lequel la fonction sigmoïde $f_{15}$ comprend une série de fonctions sigmoïdes avec des régions distinctement différentes de fractions extractibles de manière homogène, conformément à :

$$\frac{df_i}{dt} = \sum_{i=1}^{n} \frac{df_i}{dt} = \sum_{i=1}^{n} k_{ei}(a_i - f_i), \sum_{i=1}^{n} a_i = 1, f_i \leq a_i$$

où :

$f_i$ = la ième fraction de PHA extrait (0 < fi < ai) ;
$k_{ei}$ = la constante de vitesse d'extraction de 1er ordre de la ième fraction.

9. Procédé selon la revendication 1, comportant :

- le mélange de la biomasse avec un solvant pauvre en PHA dans le réacteur ;
- avant l'introduction de la biomasse dans le réacteur, la formation de la biomasse en granulés avec les granulés présentant une répartition granulométrique avec D50 entre 0,1 et 4 mm ;

dans lequel le PHA dans la biomasse acheminée vers le réacteur est thermiquement stable et comporte une température de décomposition thermique supérieure à 270 °C ; et

- après l'extraction du PHA à partir de la biomasse contenant des PHA, la séparation du solvant riche en PHA de la biomasse et le maintien du solvant pour PHA à une température suffisante pour empêcher une gélification lors du procédé de séparation.

10. Procédé selon la revendication 1, dans lequel, pendant l'étape d'extraction du PHA à partir de la biomasse contenant des PHA, la fourniture d'un procédé de surveillance en ligne pour surveiller la cinétique et l'avancement de l'extraction du PHA, dans lequel le procédé de surveillance en ligne est utilisé pour contrôler le temps d'extraction pour obtenir une solution riche en PHA.

11. Procédé selon une quelconque revendication précédente, dans lequel le procédé comprend le réglage précis et en temps réel du temps d'extraction d'un lot à l'autre, dans lequel le réglage précis comprend de préférence le réglage précis du temps du procédé d'extraction sur la base d'une surveillance en ligne du procédé d'extraction.

**12.** Procédé selon la revendication 10, dans lequel le procédé de surveillance en ligne comprend la mesure des changements de viscosité du solvant pauvre en PHA pendant une période de temps sélectionnée, et dans lequel la mesure comprend de préférence la mesure des changements compensés en température de la viscosité du solvant pauvre en PHA pendant une période de temps sélectionnée.

**13.** Procédé selon la revendication 10, dans lequel le procédé de surveillance en ligne comprend la surveillance du solvant pauvre en PHA par spectroscopie proche infrarouge (NIR) ou comprend la surveillance des évolutions de la pression en fonction de la température dans un récipient d'extraction.

**14.** Procédé selon la revendication 1, comprenant la sélection d'une charge de biomasse dans le système d'extraction sur la base d'un essai à l'échelle du laboratoire pour déterminer le taux de scission spécifique tel que décrit dans la description, pour que la masse moléculaire moyenne en poids du PHA récupéré soit au moins de 350 kDA, de préférence supérieure à 500 kDa, de manière davantage préférée supérieure à 700 kDa.

**15.** Procédé de récupération de PHA à partir d'une biomasse de culture mélangée comprenant :

a. la préparation d'une biomasse riche en PHA sous forme de granulés ;
b. la détermination d'une tendance d'extraction isotherme en fonction de la température ;
c. la modélisation empirique de la tendance d'extraction isotherme en une fonction sigmoïde ($f_{15}$) qui décrit la tendance de l'augmentation du niveau de polymère extrait avec la température après 15 minutes conformément à :

$$f_{15} = \frac{1}{1 + e^{-k_{15}(T - T_H)}}$$

où

$f_{15}$ = fraction de polymère extractible après 15 minutes en fonction de la température (T) ;
$k_{15}$ = coefficient de température de vitesse d'extraction de 15 minutes ;
$T_H$ = la température pour $F_{15}$ égale à 0,50 ;

d. la détermination, à partir de la fonction sigmoïde ($f_{15}$), d'une température limitante inférieure $TL_{15}$ et d'une température limitante supérieure $TU_{15}$, dans lequel la température limitante inférieure $TL_{15}$ est une température en dessous de laquelle une extraction négligeable se produira en 15 minutes et la température limitante supérieure $TU_{15}$ est une température à laquelle une extraction importante se produira en 15 minutes ;
e. le mélange de la biomasse riche en PHA sous forme de granulés et d'un solvant pauvre en PHA ;
f. l'extraction du PHA à partir de la biomasse riche en PHA pour produire un solvant riche en PHA par la dissolution du PHA dans un solvant pauvre en PHA à des températures supérieures à la température limitante d'extraction ($TL_{15}$) ;
g. pendant l'extraction, le maintien de la température du solvant pauvre en PHA pour que la température, en moyenne, soit entre $TL_{15}$ et $TU_{15}$ pendant une période de temps supérieure à 15 minutes, de préférence inférieure à 1 heure et de manière préférée entre toutes inférieure à 2 heures ;
h. après l'extraction du PHA à partir de la biomasse riche en PHA, la séparation du solvant riche en PHA de la biomasse résiduelle tout en maintenant la température au-dessus d'une température de gélification ($T_{gel}$) ;
i. le transfert du solvant riche en PHA vers un emplacement de gélification tout en maintenant la température de la température ($T_{gel}$) du solvant riche en PHA à une température qui empêche une gélification ;
j. le fait de favoriser la gélification du solvant riche en PHA à un emplacement prédéterminé dans le procédé par le refroidissement du solvant riche en PHA jusqu'à une température inférieure ou égale à la température de gélification avec ou sans mélange ; et
k. l'élimination par pressage du solvant à partir du gel de solvant riche en PHA.

*Figure 1.*

*Figure 2.*

*Figure 3.*

*Figure 4.*

*Figure 5.*

Figure 6.

*Figure 7.*

*Figure 8.*

*Figure 9. Particle size distribution of a PHA-rich biomass after roll-mill crushing showing the estimated distribution passing a 2.00 mm mesh and being retained by a 0.71 mm mesh.*

*Figure 10.*

*Figure 11.*

Figure 12.

*Figure 13.*

*Figure 14.*

*Figure 15.*

*Figure 16.*

*Figure 17.*

Figure 18.

*Figure 19.*

*Figure 20.*

*Figure 21.*

*Figure 22.*

*Figure 23.*

*Figure 24.*

*Figure 25.*

*Figure 26.*

*Figure 27.*

Figure 28.

*Figure 29.*

*Figure 30.*

*Figure 31.*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2012022998 A1
- US 6087471 A
- US 4360488 A
- US 7226765 B
- WO 9606179 A1
- WO 2011070544 A2